# EUROPEAN PATENT APPLICATION

(11) **EP 3 546 477 A1**
(43) Date of publication of application: **02.10.2019**
(21) Application number: 19169019.7
(22) Date of filing: 13.02.2015
(51) Int. Cl.: C07K 14/47, A61K 38/17, A61P 35/00

(54) **POLYPEPTIDE FRAGMENTS OF 168A-T2 AND COMPOSITIONS COMPRISING THEM FOR USE IN TREATING CANCER**

(30) Priority: 14.02.2014 EP 14155243; 14.02.2014 US 201461939932 P
(62) Divisional of application: 15704545.1
(71) Applicant: Gene Signal International SA, 1015 Lausanne (CH)
(72) Inventor: AL-MAHMOOD, Salman, deceased (FR); COLIN, Sylvie, 94230 Cachan (FR)
(74) Representative: Icosa

(57) **Abstract**

The present invention relates to specific polypeptides having anti-angiogenic activity and their use for treating cancer.

## Description

### FIELD OF INVENTION

The present invention relates to the field of treatments for angiogenesis-related diseases, and more particularly cancers and/or tumors. The present invention more specifically relates to polypeptides possessing an anti-angiogenic activity for treating cancers and/or tumors wherein cells are expressing CD9 partner 1 (CD9P-1).

### BACKGROUND OF INVENTION

Angiogenesis corresponds to the process allowing new blood vessels to be formed from pre-existing vessels. Angiogenesis begins with the activation of endothelial cells and includes endothelial cell migration, proliferation and differentiation into capillaries. Whereas angiogenesis is a normal and vital process in growth, development and wound healing, this process may also play a key role in the progression of cancers and tumors. Indeed, the formation of new blood vessels is often stimulated by signaling molecules released by tumors, thereby enhancing the newly formed network of vessels for supplying nutrients and oxygen and removing waste products. Tumor-induced angiogenesis thus leads to tumor growth and possibly to metastasizing thereof to distant sites. Consequently, the development of anti-angiogenesis compounds has become a major focus in cancer drug development. Several chemotherapeutic molecules targeting angiogenesis are already available on the market. Among them, well known as angiogenesis inhibitors comprise angiostatin, endostatin, interferons, platelet factor 4, prolactin 16 kDa fragment, thrombospondin, TIMP-1 (tissue inhibitor of metalloprotease -1), TIMP-2 and TIMP-3, as well as other drugs such as for example combrestatin A4, EMD 121974, TNP-470, squalamine, thalidomide, interferon- alpha, anti-VEGF antibodies. Nevertheless, their efficiency remains insufficient, and their use is often accompanied by deleterious secondary effects. There is therefore still a need for alternative treatments, having increased efficiency, being less invasive or toxic, and resulting in an increased rate of recovery.

WO 03/080105 describes five genes involved in the regulation of angiogenesis and more specifically the gene "168" encoding "protein 168A", also known as CD9 partner 1 "CD9P-1". WO 03/080105 in particular discloses that protein 168A is implied in the activation of angiogenesis and is expressed in endothelial cells stimulated with pro-angiogenic factors, such as for example TNF-alpha. WO 03/080105 also describes that inhibiting the expression of gene 168 results in the inhibition of the formation of capillary tubes.

WO 2008/138894 discloses various truncated forms of protein 168A, among which protein 168A-T2 corresponds to a fragment of the extracellular domain of protein 168A. WO 2008/138894 further discloses that protein 168A-T2 (hereinafter identified as SEQ ID NO: 3) may inhibit both human endothelial cell proliferation *in vitro* and capillary tube formation *in vitro,* in a dose dependent manner. Further, this application discloses that 168A-T2 may induce the inhibition of the migration of endothelial cells *in vitro*, in a dose dependent manner. According to the results disclosed in WO 2008/138894, 168A-T2 thus appears as a potent anti-angiogenic compound, at least 600-fold more potent than the anti-VEGF mAb and/or VEGF receptor (KDR) -based identified peptides. Finally, WO 2008/138894 discloses that protein 168A-T2 has a strong anti-tumor activity *in vivo,* and a strong synergistic activity in combination with other chemotherapeutic agent such as, for example, cisplatin.

Further, a treatment of NCI-H460-xenografted *Nude* mice with GS-168AT2 (a tagged form of 168A-T2) was shown to lead to a drastic inhibition of tumor growth, and to the *in vivo* downregulation of CD9 in tumors (Guilmain et al., 2011, British Journal of Cancer, 104: 496-504).

CD9 is the most studied tetraspanin: it is known to function in multiple cell events, including membrane fusion, differentiation and cell motility and seems to have a key role in metastasis. Clinical observations suggest that downregulation of CD9 is associated with the progression of solid tumors. Tetraspanins compose a family of proteins with four transmembrane domains delineating two extracellular domains of unequal size, which are involved in numerous physiological processes including angiogenesis, cell migration, cell-cell contact and fusion. The function of tetraspanins is thought to be related to their ability to interact with one another and with various other surface proteins, forming a network of molecular interactions referred to as the tetraspanin web. CD9 partner 1 (also known as "CD9P-1", "FPRP" or "EWI-F") associates with CD9, CD81 and CD151 and localizes therefore within the tetraspanins web.

It was further shown that CD9P-1 expression is essential for angiogenesis, and that the protein GS-168AT2 (a truncated form of CD9P-1) inhibits dose-dependently human endothelial cell (hEC) proliferation, migration, *in vitro* and *in vivo* angiogenesis, as well as *in vivo* tumor growth. It was in particular proposed that GS-168AT2 exerted its action by downregulating CD9 and CD151 at the cell surface, in reducing the expression of CD9P-1 (Colin et al., 2011, British Journal of Cancer, 105:1002-1011).

168A-T2 was shown to induce strong perturbations of the tetraspanins web, in triggering the degradation of CD9, CD151 and CD9P-1. It is therefore of interest to provide a therapeutic solution that specifically target only one tetraspanin.

The present inventors have surprisingly discovered that two peptides (hereinafter P3 and P4, of respective SEQ ID NO: 1 and 2) comprised within the sequence of 168A-T2 actually possess on their own an anti-angiogenic activity. The data presented hereinafter thus demonstrate that these peptides may be of use either as a primary anti-tumoral agent or as an add-on synergic therapy to primary cytotoxic agents for the treatment of angiogenesis-related diseases, and more specifically for treating cancers and/or tumors. Surprisingly, the said peptides were shown to trigger the degradation of CD151 only, while leaving unchanged the cellular levels of CD9 and CD9P-1, thereby limiting the deleterious effects resulting from the destructuration of the tetraspanin web (as obtained with 168A-T2). Further, the said peptides were advantageously demonstrated to exert their inhibitory activity only when cells are under an angiogenic state (i.e. only when cells are expressing CD9P-1), as a contrary to state-of-the-art anti-CD 151 antibodies. Peptides P3 and P4 are therefore constituting unexpected and advantageous alternatives to the known-to-date cancer treatments, since they are, for instance, capable to induce the expression of endostatins, a major angiogenesis inhibitor, without the drawbacks of recombinant endostatin (difficulty to produce in large quantities, loss of biological activity upon long-term storage, administration to patients resulting in secondary effects, etc).

### SUMMARY

The present invention thus relates to a polypeptide, characterized in that it comprises at least one peptide selected from the group consisting in:
(i) a peptide of sequence GNYYCSVTPWVKS (SEQ ID NO: 1);
(ii) a peptide of sequence IHSKPVFITVKMDVLNA (SEQ ID NO: 2); and
(iii) a functional fragment or a variant or derivative thereof;
wherein said polypeptide comprises less than 50, preferably less than 40, preferably less than 30, preferably less than 20 contiguous amino acids of a SEQ ID NO: 3.

In one embodiment, the polypeptide of the invention has an anti-angiogenic activity.

In a particular embodiment, the polypeptide of the invention comprises both a peptide of SEQ ID NO: 1 and a peptide of SEQ ID NO: 2. In a specific embodiment of the invention, the polypeptide consists in a peptide of SEQ ID NO: 1. In another specific embodiment of the invention, the polypeptide consists in a peptide of SEQ ID NO: 2.

In an embodiment of the invention, the said polypeptide is modified by the addition of one or more functional group such as a phosphate, acetate, lipid or carbohydrate group, and/or by the addition of one or more protecting group.

In an embodiment, the said polypeptide further possesses anti-tumoral activity.

The present invention further concerns a polynucleotide encoding a polypeptide as defined above.

The present invention further concerns a pharmaceutical composition comprising a polypeptide or a polynucleotide as define above.

In an embodiment, the pharmaceutical composition of the invention further comprises at least one pharmaceutically acceptable excipient.

The present invention further concerns a pharmaceutical composition comprising a polypeptide or a polynucleotide as define above and at least one pharmaceutically acceptable excipient.

In an embodiment, the pharmaceutical composition of the invention further comprises at least one cytotoxic, chemotherapeutic or anti-cancer agent. In a particular embodiment of the invention, the pharmaceutical composition further comprises a platinum complex selected form the group consisting of cisplatin.

In an embodiment, the pharmaceutical composition of the invention further comprises at least one anti-angiogenic agent.

In a particular embodiment, the pharmaceutical composition of the invention comprises:
- a polypeptide comprising a peptide of SEQ ID NO: 1 and a peptide of SEQ ID NO: 2; or
- a first polypeptide comprising a peptide of SEQ ID NO: 1, and a second polypeptide comprising a peptide of SEQ ID NO: 2.

In a particular embodiment, the pharmaceutical composition of the invention comprises a polypeptide consisting in a peptide of SEQ ID NO: 1. In another specific embodiment of the invention, the pharmaceutical composition of the invention comprises a polypeptide consisting in a peptide of SEQ ID NO: 2.

In an embodiment, the pharmaceutical composition of the invention is in the form suitable for topical, systemic, oral, subcutaneous, transdermal, intramuscular or intra-peritoneal administration.

The invention further concerns a polypeptide or a pharmaceutical composition according to the invention for use in the treatment of an angiogenesis-related disease.

In an embodiment, the invention concerns a polypeptide or a pharmaceutical composition according to the invention for use in the treatment of cancer and/or of tumors in the human or animal body.

In an embodiment, the invention concerns a polypeptide or a pharmaceutical composition according to the invention for use in the treatment of a cancer and/or tumor wherein cancer cells express CD9P-1.

In an embodiment, the invention concerns a polypeptide or pharmaceutical composition for use in the treatment of a cancer and/or tumor in a subject in need thereof wherein cancer cells express CD9P-1, wherein prior to the treatment, the expression of CD9P-1 is tested in a sample comprising cancer and/or tumor cells of the subject.

### DETAILED DESCRIPTION

This invention thus relates to a polypeptide characterized in that said polypeptide comprises or consists in the peptide sequence GNYYCSVTPWVKS (P3 - SEQ ID NO: 1); IHSKPVFITVKMDVLNA (P4 - SEQ ID NO: 2); or is a functional fragment or variant or derivative thereof; and wherein said polypeptide comprises less than 50, 45, 40, 35, 30, 25, 20, 19, 18, 17, 16, 15, 14, 13 contiguous amino acids of a SEQ ID NO: 3.

In one embodiment, the polypeptide of the invention has an anti-angiogenic activity.

As used herein, the term "functional fragment" of SEQ ID NO: 1 or SEQ ID NO: 2 or of a polypeptide comprising SEQ ID NO: 1 or SEQ ID NO: 2 refers to a fragment of SEQ ID NO: 1 or SEQ ID NO: 2 or of a polypeptide comprising SEQ ID NO: 1 or SEQ ID NO: 2 that has the same function as SEQ ID NO: 1 or SEQ ID NO: 2, preferably that has the same anti-angiogenic activity of SEQ ID NO: 1 or SEQ ID NO: 2.

As used herein, the term "polypeptide" means molecules formed from the linking, in a defined order, of amino acids, and of at least 10, 15, 20, 25, 50, 75 to 100 amino acids. An "isolated" polypeptide according to the invention refers to one that has been removed from its natural environment or to one that has been designed by a person skilled in the art.

In one embodiment, the polypeptide according to the invention has a length of at least 13, 14, 15, 16, 17, 18, 19, 20, 21, 22, 23, 24, 25, 26, 27, 28, 29, 30, 35, 40, 45, 50, 60, 70, 75, 80, 90 or 100 amino acids.

In one embodiment, the polypeptide of the invention comprises (or consists in) from 13 to 50 amino acids. In another embodiment of the invention, the polypeptide comprises from 13 to 40 amino acids. In another embodiment of the invention, the polypeptide comprises (or consists in) from 13 to 30 amino acids. In another embodiment of the invention, the polypeptide comprises (or consists in) from 13 to 25, 24, 23, 22, or 21 amino acids. In another embodiment of the invention, the polypeptide comprises (or consists in) from 13 to 20 amino acids. In another embodiment of the invention, the polypeptide comprises (or consists in) from 13 to 19, 18, 17, 16, 15 or 14 amino acids. In another embodiment of the invention, the polypeptide comprises (or consists in) 13 amino acids.

In another embodiment, the polypeptide of the invention comprises (or consists in) from 17 to 50 amino acids. In another embodiment of the invention, the polypeptide comprises (or consists in) from 17 to 40 amino acids. In another embodiment of the invention, the polypeptide comprises (or consists in) from 17 to 30 amino acids. In another embodiment of the invention, the polypeptide comprises (or consists in) from 17 to 25, 24, 23, 22 or 21 amino acids. In another embodiment of the invention, the polypeptide comprises (or consists in) from 17 to 20, 19 or 18 amino acids.

In one embodiment of the invention, said peptide has 50, 40, 30, 25, 20, 19, 18, 17, 16, 15, 14, 13 amino acids length and comprises the peptide sequence SEQ ID NO: 1, wherein said polypeptide comprises less than 50, 45, 40, 35, 30, 25, 20, 19, 18, 17, 16, 15, 14, 13 contiguous amino acids of a SEQ ID NO: 3.

In another embodiment of the invention, said peptide has 50, 40, 30, 25, 22, 20, 19, 18, 17 amino acids length and comprises the peptide sequence SEQ ID NO: 2, wherein said polypeptide comprises less than 50, 45, 40, 35, 30, 25, 20, 19, 18, 17 contiguous amino acids of a SEQ ID NO: 3.

In one embodiment, the polypeptide of the invention comprises SEQ ID NO: 1 and 1, 2, 3, 4, 5, 6, 7, 8, 9, 10 or more amino acids in C-term, and/or 1, 2, 3, 4, 5, 6, 7, 8, 9, 10 or more amino acids in N-term.

As used herein, "amino acids" are represented by their full name, their three letter code or their one letter code as well known in the art. Amino acid residues in peptides are abbreviated as follows: Phenylalanine is Phe or F; Leucine is Leu or L; Isoleucine is Ile or I; Methionine is Met or M; Valine is Val or V; Serine is Ser or S; Proline is Pro or P; Threonine is Thr or T; Alanine is Ala or A; Tyrosine is Tyr or Y; Histidine is His or H; Glutamine is Gln or Q; Asparagine is Asn or N; Lysine is Lys or K; Aspartic Acid is Asp or D; Glutamic Acid is Glu or E; Cysteine is Cys or C; Tryptophan is Trp or W; Arginine is Arg or R; and Glycine is Gly or G.

As used herein, the term "amino acids" includes both natural and synthetic amino acids, and both D and L amino acids. "Standard amino acid" or "naturally occurring amino acid" means any of the twenty standard L-amino acids commonly found in naturally occurring peptides. "Nonstandard amino acid residue" means any amino acid, other than the standard amino acids, regardless of whether it is prepared synthetically or derived from a natural source. For example, naphtlylalanine can be substituted for tryptophan to facilitate synthesis. Other synthetic amino acids that can be substituted include, but are not limited to, L-hydroxypropyl, L-3,4-dihydroxyphenylalanyl, alpha-amino acids such as L-alpha-hydroxylysyl and D-alpha-methylalanyl, L-alpha-methylalanyl, beta-amino acids, and isoquinolyl.

As used herein, "amino acid" also encompasses chemically modified amino acids, including but not limited to salts, amino acid derivatives (such as amides), and substitutions. Amino acids contained within the polypeptides of the present invention, and particularly at the carboxy- or amino-terminus, can be modified by methylation, amidation, acetylation or substitution with other chemical groups which can change the polypeptide's circulating half-life without adversely affecting their activity. Additionally, a disulfide linkage may be present or absent in the polypeptides of the invention.

The polypeptides of the invention may comprise naturally standard amino acids or nonstandard amino acids. Polypeptide mimetics include polypeptides having the following modifications: i) polypeptides wherein one or more of the peptidyl -C(O)NR- linkages (bonds) have been replaced by a non-peptidyl linkage such as a -CH₂-carbamate linkage (-CH₂OC(O)NR-), a phosphonate linkage, a -CH₂-sulfonamide (-CH₂-S(O)₂NR-) linkage, a urea (-NHC(O)NH-) linkage, a -CH₂-secondary amine linkage, or with an alkylated peptidyl linkage (-C(O)NR-) wherein R is C₁-C₄ alkyl; ii) polypeptides wherein the N-terminus is derivatized to a -NRR¹ group, to a -NRC(O)R group, to a -NRC(O)OR group, to a -NRS(O)₂R group, to a -NHC(O)NHR group where R and R¹ are hydrogen or C₁-C₄ alkyl with the proviso that R and R¹ are not both hydrogen; iii) polypeptides wherein the C terminus is derivatized to -C(O)R² where R² is selected from the group consisting of C₁-C₄ alkoxy, and -NR³R⁴ where R³ and R⁴ are independently selected from the group consisting of hydrogen and C₁-C₄ alkyl.

In one embodiment, the polypeptide of the invention comprises SEQ ID NO: 1 or SEQ ID NO: 2 or a variant thereof. In one embodiment, a variant of SEQ ID NO: 1 or SEQ ID NO: 2 has an anti-angiogenic activity, preferably an anti-angiogenic activity equivalent to the one of SEQ ID NO: 1 or SEQ ID NO: 2.

In one embodiment, a variant of SEQ ID NO: 1 or SEQ ID NO: 2 comprises conservative amino acid substitutions as compared to the sequence of SEQ ID NO: 1 or SEQ ID NO: 2 respectively.

As used herein, the term "conservative amino acid substitution" is defined herein as an amino acid exchange within one of the following five groups:
I. Small aliphatic, nonpolar or slightly polar residues: Ala, Ser, Thr, Pro, Gly;
II. Polar, negatively charged residues and their amides: Asp, Asn, Glu, Gln;
III. Polar, positively charged residues: His, Arg, Lys;
IV. Large, aliphatic, nonpolar residues: Met, Leu, Ile, Val, Cys
V. Large, aromatic residues: Phe, Tyr, Trp.

In another embodiment, a variant of SEQ ID NO: 1 or SEQ ID NO: 2 is a polypeptide having a sequence identity of at least 70%, preferably of at least 75, 80, 85, 90, 95, 96, 97, 98, 99% or more with SEQ ID NO: 1 or SEQ ID NO: 2 respectively.

In another embodiment, a variant of SEQ ID NO: 1 or SEQ ID NO: 2 is a polypeptide wherein 1, 2, 3, 4, or 5 amino acids from the sequence of SEQ ID NO: 1 or SEQ ID NO: 2 respectively is/are absent, or substituted by any amino acid, or wherein 1, 2, 3, 4 or 5 amino acids (either contiguous or not) is/are added.

The term "identity" or "identical", when used in a relationship between the sequences of two or more polypeptides, refers to the degree of sequence relatedness between polypeptides, as determined by the number of matches between strings of two or more amino acid residues. "Identity" measures the percent of identical matches between the smaller of two or more sequences with gap alignments (if any) addressed by a particular mathematical model or computer program (i.e., "algorithms"). Identity of related polypeptides can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48,1073 (1988). Preferred methods for determining identity are designed to give the largest match between the sequences tested. Methods of determining identity are described in publicly available computer programs. Preferred computer program methods for determining identity between two sequences include the GCG program package, including GAP (Devereux et al., Nucl. Acid. Res. \2, 387 (1984); Genetics Computer Group, University of Wisconsin, Madison, Wis.), BLASTP, BLASTN, and FASTA (Altschul et al., J. Mol. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity.

The polypeptides described herein can be produced synthetically by chemical synthesis or enzymatic synthesis as it is well known in the art. Alternatively, nucleotide sequences encoding the polypeptides of the invention can be introduced into a protein expression vector and produced in a suitable host organism (e.g., bacteria, insect cells, etc), then purified. An additional polypeptide ("tag") can be added on for the purpose of purifying or identifying or purifying the polypeptides. Protein tags make it possible, for example, for the polypeptides to be adsorbed, with high affinity, to a matrix, and for the matrix then to be washed stringently with suitable buffers without the complex being eluted to any significant extent, and for the adsorbed complex subsequently to be eluted selectively. Examples of protein tags which are known to the skilled person are a (His)₆ tag, a Myc tag, a FLAG tag, a haemagglutinin tag, a glutathione transferase (GST) tag, intein having an affinity chitin- binding tag or maltose-binding protein (MBP) tag. These protein tags can be located N- terminally, C -terminally and/or internally.

In one embodiment of the invention, the polypeptides as described here above are modified by means well-known in the art, for instance by the addition of one or more functional group such as a phosphate, acetate, lipid or carbohydrate group, and/or by the addition of one or more protecting group.

For example, the polypeptides can be modified by the addition of one or more functional groups such as phosphate, acetate, or various lipids and carbohydrates. The polypeptides of the invention can also exist as polypeptide derivatives. The term "polypeptide derivative" refers to compound having an amino group (--NH--), and more particularly, a peptide bond. Polypeptides may be regarded as substituted amides. Like the amide group, the peptide bond shows a high degree of resonance stabilization. The C--N single bond in the peptide linkage has typically about 40 percent double-bond character and the C=O double bond about 40 percent single-bond character. "Protecting groups" are those groups that prevent undesirable reactions (such as proteolysis) involving unprotected functional groups. Specific examples of amino protecting groups include formyl; trifluoroacetyl; benzyloxycarbonyl; substituted benzyloxycarbonyl such as (ortho- or para-) chlorobenzyloxycarbonyl and (ortho- or para-) bromobenzyloxycarbonyl; and aliphatic oxycarbonyl such as t-butoxycarbonyl and t-amiloxycarbonyl. The carboxyl groups of amino acids can be protected through conversion into ester groups. The ester groups include benzyl esters, substituted benzyl esters such as methoxybenzyl ester; alkyl esters such as cyclohexyl ester, cycloheptyl ester or t-butyl ester. The guanidino moiety may be protected by nitro; or arylsulfonyl such as tosyl, methoxybenzensulfonyl or mesitylenesulfonyl, even though it does not need a protecting group. The protecting groups of imidazole include tosy, benzyl and dinitrophenyl. The indole group of tryptophan may be protected by formyl or may not be protected.

The modification of the polypeptides aims in particular to improve their life time *in vivo.* One type of modification is the addition to the N or C termini of the polypeptides of polyethylene glycol (PEG). PEG is known by the person skilled in the art to have many properties that make it an ideal carrier for polypeptides such as high water solubility, high mobility in solution and low immunogenicity. This modification also protects the polypeptides from exopeptidases and therefore increases their overall stability *in vivo.*

The other modifications used to prevent degradation of the polypeptides by endopeptidases or exopeptidases include N-terminal modifications such as acetylation or glycosylation, C-terminal modifications such as amidation and use of unnatural amino acids (β-amino and α-trifluoromethyl amino acids) at particular sites within the polypeptides.

Another alternative to increase polypeptide molecular size is the genetic fusion of the polypeptides to the Fc domain of human gamma immunoglobulin or the fusion of the polypeptides to albumin.

In a particular embodiment, the polypeptide of the invention comprises both a peptide of SEQ ID NO: 1 and a peptide of SEQ ID NO: 2. In one embodiment, the polypeptide of the invention comprises a peptide of SEQ ID NO: 1 fused to a peptide of SEQ ID NO: 2. In one embodiment, the polypeptide of the invention comprises or consists in SEQ ID NO: 1 and SEQ ID NO: 2 separated by a linker, corresponding to an amino acid sequence of 1, 2, 3, 4, 5, 6, 7, 8, 9, 10, 11, 12, 13, 14, 15, 16, 17, 18, 19, 20 or more amino acids. In one embodiment, SEQ ID NO: 1 and SEQ ID NO: 2 are in the same orientation in the polypeptide of the invention. In another embodiment, SEQ ID NO: 1 and SEQ ID NO: 2 are in opposite orientations in the polypeptide of the invention.

In another specific embodiment of the invention, the polypeptide consists in a peptide of SEQ ID NO: 1. In another specific embodiment of the invention, the polypeptide consists in a peptide of SEQ ID NO: 2.

In one embodiment, the polypeptide of the invention consists in a peptide of SEQ ID NO: 1 or 2 and is pegylated.

Another object of the invention is a composition comprising at least one polypeptide of the invention.

Another object of the invention is a pharmaceutical composition comprising at least one of the polypeptides as described here above in combination with pharmaceutically acceptable excipients.

Another object of the invention is a medicament comprising at least one polypeptide of the invention.

The term "pharmaceutically acceptable" refers to compounds and compositions which may be administered to mammals without undue toxicity. Accordingly, a "pharmaceutically acceptable excipient" refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet sterility, pyrogenicity, general safety and purity standards as required by regulatory offices, such as, for example, FDA Office or EMA.

Suitable excipients include water, saline, Ringer's solution, dextrose solution, and solutions of ethanol, glucose, sucrose, dextran, mannose, mannitol, sorbitol, polyethylene glycol (PEG), phosphate, acetate, gelatin, collagen, Carbopol®, vegetable oils, and the like. One may additionally include suitable preservatives, stabilizers, antioxidants, antimicrobials, and buffering agents, such as, for example, BHA, BHT, citric acid, ascorbic acid, tetracycline, and the like.

Other examples of pharmaceutically acceptable excipients that may be used in the composition of the invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene- polyoxypropylene- block polymers, polyethylene glycol and wool fat.

In one embodiment, the composition, pharmaceutical composition or medicament of the invention may comprise some excipients, such as, for example, surfactants (e.g. hydroxypropylcellulose); suitable carriers, such as, for example, solvents and dispersion media containing, for example, water, ethanol, polyol (e.g. glycerol, propylene glycol, and liquid polyethylene glycol, and the like), suitable mixtures thereof, and vegetable oils, such as, for example, peanut oil and sesame oil; isotonic agents, such as, for example, sugars or sodium chloride; coating agents, such as, for example, lecithin; agents delaying absorption, such as, for example, aluminum monostearate and gelatin; preservatives, such as, for example, benzalkonium chloride, benzethonium chloride, chlorobutanol, thimerosal and the like; buffers, such as, for example, boric acid, sodium and potassium bicarbonate, sodium and potassium borates, sodium and potassium carbonate, sodium acetate, sodium biphosphate and the like; tonicity agents, such as, for example, dextran 40, dextran 70, dextrose, glycerin, potassium chloride, propylene glycol, sodium chloride; antioxidants and stabilizers, such as, for example, sodium bisulfite, sodium metabisulfite, sodium thiosulfite, thiourea and the like; nonionic wetting or clarifying agents, such as, for example, polysorbate 80, polysorbate 20, poloxamer 282 and tyloxapol; viscosity modifying agents, such as, for example dextran 40, dextran 70, gelatin, glycerin, hydroxyethylcellulose, ydroxmethylpropylcellulose, lanolin, methylcellulose, petrolatum, polyethylene glycol, polyvinyl alcohol, polyvinylpyrrolidone, carboxymethylcellulose; and the like.

In one embodiment, the composition, pharmaceutical composition or medicament may comprise a pharmaceutically acceptable salt of the polypeptide.

Examples of the pharmaceutically acceptable salt include salts with inorganic bases, salts with organic bases, salts with inorganic acids, salts with organic acids, salts with basic or acidic amino acids and the like. Examples of the salt with an inorganic base include alkali metal salts, such as a sodium salt and a potassium salt; an alkaline earth metal salt such as a calcium salt and a magnesium salt; an aluminum salt; and an ammonium salt. Examples of the salt with an organic base include salts with trimethylamine, triethylamine, pyridine, picoline, 2,6-lutidine, ethanolamine, diethanolamine, triethanolamine, cyclohexylamine, dicyclohexylamine and N,N'-dibenzylethylenediamine. Examples of the salt with an inorganic acid include salts with hydrochloric acid, boric acid, nitric acid, sulfuric acid and phosphoric acid. Examples of the salt with an organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid and p-toluenesulfonic acid. Examples of the salt with a basic amino acid include salts with arginine, lysine and ornithine. Examples of the salt with an acidic amino acid include salts with aspartic acid and glutamic acid. The list of suitable salts is disclosed in Remington's Pharmaceutical Sciences, 17th ed., Mack Publishing Company, Easton, Pa., p 1418, 1985, the entire disclosure of which is incorporated herein by reference.

In one embodiment, the amount of polypeptide present in the medicament, composition or pharmaceutical composition is effective to treat an angiogenesis-related disease, preferably to treat cancer and/or tumors and/or susceptible cancers and/or susceptible tumors. Preferably, the polypeptide is present in an amount from 0.01 to 90% in weight, preferably from 0.1% to 10% in weight, more preferably from 1% to 5% in weight, in the medicament or in the composition. These amounts are routinely adaptable by the man in the art, who is able to choose the best quantity to administer to a patient to achieve recovery.

In one embodiment, the composition, pharmaceutical composition or medicament of the invention comprises at least 10 mg of the polypeptide as described hereinabove, preferably at least 20 mg, more preferably at least 50 mg. In a preferred embodiment, the composition , pharmaceutical composition or medicament comprises from 10 to 3000 mg of the polypeptide of the invention, preferably from 50 to 2000 mg, more preferably from 100 to 1500 mg.

In a particular embodiment, the composition, pharmaceutical composition or medicament of the invention comprises a polypeptide consisting in a peptide of SEQ ID NO: 1. In another specific embodiment of the invention, the composition, pharmaceutical composition or medicament of the invention comprises a polypeptide consisting in a peptide of SEQ ID NO: 2. In another specific embodiment of the invention, the composition, pharmaceutical composition or medicament of the invention comprises a polypeptide consisting in a peptide of SEQ ID NO: 1 or 2, said peptide being pegylated.

In one embodiment, the composition, pharmaceutical composition or medicament of the invention comprises a polypeptide as described hereinabove, wherein said polypeptide comprises two peptides, a peptide of SEQ ID NO: 1, and a peptide of SEQ ID NO: 2.

In another embodiment, the composition, pharmaceutical composition or medicament of the invention comprises two polypeptides, a first polypeptide comprising a peptide of SEQ ID NO: 1, and a second polypeptide comprising a peptide of SEQ ID NO: 2. In another embodiment, the composition, pharmaceutical composition or medicament of the invention comprises two polypeptides, a first polypeptide consisting of a peptide of SEQ ID NO: 1, and a second polypeptide consisting of a peptide of SEQ ID NO: 2.

In one embodiment, the polypeptide of the invention is the only one active ingredient comprised in the composition, pharmaceutical composition or medicament of the invention.

In another embodiment, the composition, pharmaceutical composition or medicament of the invention further comprise another active ingredient, and the polypeptide of the invention may thus be an add-on therapeutic agent to said another active ingredient.

In a particular embodiment of the invention, the composition, pharmaceutical composition or medicament further comprises a cytotoxic, chemotherapeutic or anti-cancer agent.

Examples of anti-cancer agents include, but are not limited to, alkylating agents or agents with an alkylating action, such as, for example, cyclophosphamide (CTX; e.g. CYTOXAN®), chlorambucil (CHL; e.g. LEUKERAN®), cisplatin (CisP; e.g. PLATINOL®), oxaliplatin (e.g. ELOXATIN™), busulfan (e.g. MYLERAN®), melphalan, carmustine (BCNU), streptozotocin, triethylenemelamine (TEM), mitomycin C, and the like; anti-metabolites, such as, for example, methotrexate (MTX), etoposide (VP16; e.g. VEPESID®), 6-mercaptopurine (6MP), 6- thiocguanine (6TG), cytarabine (Ara-C), 5-fluorouracil (5-FU), capecitabine (e.g. XELODA®), dacarbazine (DTIC), and the like; antibiotics, such as, for example, actinomycin D, doxorubicin (DXR; e.g. ADRIAMYCIN®), daunorubicin (daunomycin), bleomycin, mithramycin and the like; alkaloids, such as, for example, vinca alkaloids such as, for example, vincristine (VCR), vinblastine, and the like; and other antitumor agents, such as, for example, paclitaxel (e.g. TAXOL®) and paclitaxel derivatives, cytostatic agents, glucocorticoids such as dexamethasone (DEX; e.g. DECADRON®) and corticosteroids such as, for example, prednisone, nucleoside enzyme inhibitors such as, for example, hydroxyurea, amino acid depleting enzymes such as, for example, asparaginase, leucovorin, folinic acid, raltitrexed, and other folic acid derivatives, and similar, diverse antitumor agents. The following agents may also be used as additional agents: amifostine (e.g. ETHYOL®), dactinomycin, mechlorethamine (nitrogen mustard), streptozocin, cyclophosphamide, lornustine (CCNU), doxorubicin lipo (e.g. DOXIL®), gemcitabine (e.g. GEMZAR®), daunorubicin lipo (e.g. DAUNOXOME®), procarbazine, mitomycin, docetaxel (e.g. TAXOTERE®), aldesleukin, carboplatin, cladribine, camptothecin, 10-hydroxy 7-ethyl-camptothecin (SN38), floxuridine, fludarabine, ifosfamide, idarubicin, mesna, interferon alpha, interferon beta, mitoxantrone, topotecan, leuprolide, megestrol, melphalan, mercaptopurine, plicamycin, mitotane, pegaspargase, pentostatin, pipobroman, plicamycin, tamoxifen, teniposide, testolactone, thioguanine, thiotepa, uracil mustard, vinorelbine, or chlorambucil.

In a preferred embodiment, the pharmaceutical composition further comprises a platinum complex. Said platinum complex may be selected form the group consisting of cisplatin, carboplatin, oxaliplatin, satraplatin, picoplatin, nedaplatin, triplatin, lipoplatin and combination thereof.

In one embodiment, the pharmaceutical composition of the invention comprises at least one polypeptide according to the invention and cisplatin. In another embodiment, the pharmaceutical composition of the invention comprises at least one polypeptide according to the invention and carboplatin.

In a preferred embodiment, the pharmaceutical composition of the invention comprises a polypeptide of SEQ ID NO: 1 (P3) and cisplatin. In another embodiment, the pharmaceutical composition of the invention comprises a polypeptide of SEQ ID NO: 2 (P4) and cisplatin. In another embodiment, the pharmaceutical composition of the invention comprises a polypeptide of SEQ ID NO: 1 (P3), a polypeptide of SEQ ID NO: 2 (P4), and cisplatin. In another embodiment, the pharmaceutical composition of the invention comprises a polypeptide of SEQ ID NO: 1 (P3) and carboplatin. In another embodiment, the pharmaceutical composition of the invention comprises a polypeptide of SEQ ID NO: 2 (P4) and carboplatin. In another embodiment, the pharmaceutical composition of the invention a polypeptide of SEQ ID NO: 1 (P3), a polypeptide of SEQ ID NO: 2 (P4), and carboplatin.

In another embodiment of the invention, the pharmaceutical composition further comprises an antiangiogenic agent. Examples of antiangiogenic agents include, but are not limited to, anti-VEGF-A, anti-VEGFR (VEGFR-1, VEGFR-2 or VEGFR-3), anti-PDGFR (α or β), anti-EGFR, anti-c-KIT. In one embodiment, said antiangiogenic agent may be selected form the group consisting of bevacizumab, ranibizumab, pegaptanib, VEGF-trap, axitinib, imatinib, vatalanib, sorafenib, semaxanib, sunitinib, vandetanib, angiostatin, endostatin, thrombospondin-1 and combination thereof.

Another object of the invention is a polypeptide as described here above or a pharmaceutical composition as described here above for treating an angiogenesis-related disease, preferably for treating cancer and/or of tumors in the human or animal body. In an embodiment, the invention concerns a polypeptide or a pharmaceutical composition according to the invention for use in the treatment of a cancer and/or tumor wherein cancer cells express CD9P-1.

As used herein, the term "treating" includes prophylaxis of the specific disorder or condition, or alleviation of the symptoms associated with a specific disorder or condition and/or preventing or eliminating said symptoms. In one embodiment, the term "treatment" thus refers to both therapeutic treatment and prophylactic or preventative measures; wherein the object is to prevent or slow down (lessen) the targeted pathologic condition or disorder. Those in need of treatment include those already with the disorder as well as those prone to have the disorder or those in whom the disorder is to be prevented. A subject or mammal is successfully "treated" for an infection if, after receiving a therapeutic amount of a polypeptide according to the present invention, the patient shows observable and/or measurable reduction in or absence of one or more of the following: reduction in the number of pathogenic cells; reduction in the percent of total cells that are pathogenic; and/or relief to some extent, of one or more of the symptoms associated with the specific disease or condition; reduced morbidity and mortality, and improvement in quality of life issues. The above parameters for assessing successful treatment and improvement in the disease are readily measurable by routine procedures familiar to a physician.

A "prophylactic" treatment is a treatment administered to a subject who does not exhibit signs of a disease or exhibits only early signs of the disease for the purpose of decreasing the risk of developing pathology associated with the disease. A "therapeutic" treatment is a treatment administered to a subject who exhibits signs of pathology for the purpose of diminishing or eliminating those signs.

A "therapeutically effective amount" of a compound is that amount of compound which is sufficient to provide a beneficial effect to the subject to which the compound is administered. In one embodiment, a "therapeutically effective amount" thus relates to the amount of a polypeptide of the invention that is necessary and sufficient for slowing down or stopping the progression, aggravation, or deterioration of one or more symptoms of the disease, or condition; alleviating the symptoms of the disease or condition ; curing the disease or condition.

Another object of the invention is a method for treating an angiogenesis-related disease, preferably a cancer and/or tumor, comprising the administration to a subject in need thereof of a therapeutically effective amount of at least one of the polypeptides of the invention.

In one embodiment, the method of the invention is a method for inhibiting angiogenesis, thereby treating an angiogenesis-related disease, preferably a cancer and/or tumor. In another embodiment, the method of the invention is a method for inhibiting endothelial cells proliferation, migration and/or differentiation into capillaries, thereby treating an angiogenesis-related disease, preferably a cancer and/or tumor. In another embodiment, the method of the invention is a method for increasing endostatin production, thereby treating an angiogenesis-related disease, preferably a cancer and/or tumor.

In another embodiment, the method of the invention is a method for inhibiting tumor growth and/or limiting or decreasing tumor volume, thereby treating a cancer and/or tumor. In another embodiment, the method of the invention is a method for triggering the degradation of CD151 while leaving unchanged the cellular levels of CD9, CD9P-1 and CD81, thereby treating a cancer and/or tumor, preferably a cancer and/or tumor wherein cancer and/or tumor cells express CD9P-1. In another embodiment, the method of the invention is a method for decreasing or inhibiting interactions between CD9P-1 and CD9, and/or between CD9P-1 and CD151, and/or between CD9 and CD151, thereby treating a cancer and/or tumor, preferably a cancer and/or tumor wherein cancer and/or tumor cells express CD9P-1.

Another object of the invention is a polypeptide, a composition, a pharmaceutical composition or a medicament as described hereinabove for treating, or for use in the treatment of an angiogenesis-related disease, preferably in the treatment of cancer and/or of tumors.

In a preferred embodiment, said polypeptide, composition, pharmaceutical composition or medicament is for use in the treatment of a cancer and/or tumor wherein cancer cells express CD9P-1. In one embodiment, the expression of CD9-P1 is tested in a sample comprising cancer and/or tumor cells obtained from the subject prior to the treatment.

In one embodiment, said polypeptide, composition, pharmaceutical composition or medicament is for use in inhibiting angiogenesis, thereby treating an angiogenesis-related disease, preferably a cancer and/or tumor. In another embodiment, said polypeptide, composition or medicament is for use in inhibiting endothelial cells proliferation, migration and/or differentiation into capillaries, thereby treating an angiogenesis-related disease, preferably a cancer and/or tumor. In another embodiment, said polypeptide, composition or medicament is for use in increasing endostatin production, thereby treating an angiogenesis-related disease, preferably a cancer and/or tumor.

In another embodiment, said polypeptide, composition, pharmaceutical composition or medicament is for use in inhibiting tumor growth and/or limiting or decreasing tumor volume, thereby treating a cancer and/or tumor. In another embodiment, said polypeptide, composition or medicament is for use in triggering the degradation of CD151 while leaving unchanged the cellular levels of CD9, CD9P-1 and CD81, thereby treating a cancer and/or tumor, preferably a cancer and/or tumor wherein cancer and/or tumor cells express CD9P-1. In another embodiment, said polypeptide, composition or medicament is for use in decreasing or inhibiting interactions between CD9P-1 and CD9, and/or between CD9P-1 and CD151, and/or between CD9 and CD151, thereby treating a cancer and/or tumor, preferably a cancer and/or tumor wherein cancer and/or tumor cells express CD9P-1.

According to the invention, the subject may be any mammal, preferably a human.

"Therapeutically effective dose or amount" refers to a dosage level sufficient to induce a desired biological result. That result can be alleviation of the signs, symptoms, or causes of a disease, or any other desired alteration of a biological system. Preferably, this dose or amount will be sufficient to alleviate the cancerous condition by killing the cancerous cells, but also to an effect that results in the inhibition of growth and/or metastasis of the cancer.

In one embodiment, the method of the invention comprises the administration of at least 10 mg of the polypeptide as described hereinabove, preferably at least 20 mg, more preferably at least 50 mg. In a preferred embodiment, the method of the invention comprises the administration of the polypeptide of the invention from 10 to 3000 mg, preferably from 50 to 2000 mg, more preferably from 100 to 1500 mg.

In one embodiment, the polypeptide, the composition, the pharmaceutical composition or the medicament for use according to the invention is administered in an amount of at least 10 mg, preferably at least 20 mg, more preferably at least 50 mg. In a preferred embodiment, the polypeptide, the composition, the pharmaceutical composition or the medicament for use according to the invention is administered in an amount from 10 to 3000 mg, preferably from 50 to 2000 mg, more preferably from 100 to 1500 mg.

In one aspect of the invention, the therapeutically effective amount of polypeptide, composition, pharmaceutical composition or medicament according to the invention ranges from about 1 to about 50 mg/kg/day, preferably from about 3 to about 40 mg/kg/day, more preferably from about 5 to about 30 mg/kg/day.

In one embodiment, the polypeptide, the composition, pharmaceutical composition or the medicament for use according to the invention is administered once or twice a day, or 1, 2, 3, 4, 5, 6 times per week. In a particular embodiment, the polypeptide, the composition or the medicament for use according to the invention is administered every day for at least 7 days, preferably at least 10 days, more preferably at least 12 days.

Cancers that may be treated by the peptides, compositions and methods of the invention include, but are not limited to:
Cardiac cancer: sarcoma (angiosarcoma, fibrosarcoma, rhabdomyosarcoma, liposarcoma), myxoma, rhabdomyoma, fibroma, lipoma and teratoma;
Lung cancer: non-small cell lung, bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatosis hamartoma, mesothelioma;
Gastrointestinal cancer: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma), colon, colorectal, rectal;
Genitourinary tract cancer: kidney (adenocarcinoma, Wihn's tumor [nephroblastoma], lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma);
Liver cancer: hepatoma (hepatocellular carcinoma), cholangiocarcinoma, hepatoblastoma, angiosarcoma, hepatocellular adenoma, hemangioma;
Bone cancer: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochondroma (osteocartilaginous exostoses), benign chondroma, chondroblastoma, chondromyxofibroma, osteoid osteoma and giant cell tumors;
Nervous system cancer: skull (osteoma, hemangioma, granuloma, xanthoma, osteitis deformans), meninges (meningioma, meningosarcoma, gliomatosis), brain (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma);
Gynecological cancer: uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma [embryonal rhabdomyosarcoma], fallopian tubes [carcinoma]);
Hematologic cancer: blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma];
Skin cancer: malignant melanoma, basal cell carcinoma, squamous cell carcinoma, Karposi's sarcoma, moles dysplastic nevi, lipoma, angioma, dermatofibroma, keloids, psoriasis; and
Adrenal glands cancer: neuroblastoma.

Cancers that may be treated by the peptides, compositions and methods of the invention include, but are not limited to: breast, prostate, colon, colorectal, lung, non-small cell lung, brain, testicular, stomach, pancreas, skin, small intestine, large intestine, throat, head and neck, oral, bone, liver, bladder, kidney, thyroid and blood.

Cancers that may be treated by the peptides, compositions and methods of the invention include: breast, prostate, colon, ovarian, colorectal, lung and non-small cell lung.

Cancers that may be treated by the peptides, compositions and methods of the invention include: breast, colon (colorectal) and lung (non-small cell lung).

Cancers that may be treated by the peptides, compositions and methods of the invention include: lymphoma and leukemia.

Cancers that may be treated by the peptides, compositions and methods of the invention include angiogenesis-related cancers such as breast carcinoma, bladder carcinomas, colon carcinomas, oral cavity tumors, advanced tumors, hairy cell leukemia, melanoma, advanced head and neck, metastatic renal cell, non-Hodgkin's lymphoma, metastatic breast, breast adenocarcinoma, advanced melanoma, pancreatic, gastric, glioblastoma, lung, ovarian, non-small cell lung, prostate, small cell lung, renal cell carcinoma, various solid tumors, multiple myeloma, metastatic prostate, malignant glioma, renal cancer, lymphoma, refractory metastatic disease, refractory multiple myeloma, cervical cancer, Kaposi's sarcoma, recurrent anaplastic glioma, and metastatic colon cancer.

Preferably, cancers that may be treated by the peptides, compositions and methods of the invention include, but are not limited to: breast cancer, carcinoid, cervical cancer, colorectal cancer, endometrial cancer, glioma, head and neck cancer, liver cancer, lung cancer, melanoma, ovarian cancer, pancreatic cancer, prostate cancer, renal cancer, skin cancer, stomach cancer, thyroid cancer and/or urothelial cancer.

In a particular embodiment, the cancer to be treated by the peptides, compositions and methods of the invention is lung cancer.

The polypeptides, compositions and methods of the invention are also intended to prevent or decrease tumor cell metastasis.

Further included within the scope of the invention is a method of treating or preventing a disease in which angiogenesis is implicated, comprising administering to a subject in need of such treatment a therapeutically effective amount of a polypeptide of the present invention.

Angiogenesis-related diseases include ocular neovascular diseases (such as, for example, ischemic retinopathy, diabetic retinopathy, retinopathy of prematurity, retinal vein occlusions, age-related macular degeneration, corneal neovascularisation, neovascular glaucoma), atherosclerosis, arthritis, psoriasis, obesity and Alzheimer's disease.

According to the invention, the polypeptides of the invention may be administered orally, topically, or by parenteral means, including subcutaneous, transdermal or intramuscular injection, implantation of sustained release depots, intravenous injection, intranasal administration, and the like.

According to the invention, the compositions comprising the polypeptides of the invention may be aqueous solutions, emulsions, creams, ointments, suspensions, gels, liposomal suspensions, and the like.

In another embodiment of the invention, the composition comprising at least one of the polypeptides of the invention may be used in combination with at least one other active ingredient for treating an angiogenesis-related disease, such as, for example, an antiangiogenic, cytotoxic, chemotherapeutic or anti-cancer agent, for providing a synergistic activity. In one aspect of the invention, the polypeptides or the composition comprising at least one of the polypeptides of the invention may be used as an add-on synergystic therapy to primary antiangiogenic, cytotoxic, chemotherapeutic or anti-cancer agents for the treatment of angiogenesis-related diseases, and more specifically for treating cancers and/or tumors.

By "synergistic", it is meant that the total effect of the combination of active principles is greater than the effect of each active principle taken separately. By "add-on synergystic therapy", it is meant a combination therapy using agents with complementary mechanisms of action that improve the therapeutic effect of a monotherapy.

Examples of anti-cancer agents include, but are not limited to, alkylating agents or agents with an alkylating action, such as, for example, cyclophosphamide (CTX; e.g. CYTOXAN®), chlorambucil (CHL; e.g. LEUKERAN®), cisplatin (CisP; e.g. PLATINOL®), oxaliplatin (e.g. ELOXATIN™), busulfan (e.g. MYLERAN®), melphalan, carmustine (BCNU), streptozotocin, triethylenemelamine (TEM), mitomycin C, and the like; anti-metabolites, such as, for example, methotrexate (MTX), etoposide (VP16; e.g. VEPESID®), 6-mercaptopurine (6MP), 6- thiocguanine (6TG), cytarabine (Ara-C), 5-fluorouracil (5-FU), capecitabine (e.g. XELODA®), dacarbazine (DTIC), and the like; antibiotics, such as, for example, actinomycin D, doxorubicin (DXR; e.g. ADRIAMYCIN®), daunorubicin (daunomycin), bleomycin, mithramycin and the like; alkaloids, such as, for example, vinca alkaloids such as, for example, vincristine (VCR), vinblastine, and the like; and other antitumor agents, such as, for example, paclitaxel (e.g. TAXOL®) and paclitaxel derivatives, the cytostatic agents, glucocorticoids such as dexamethasone (DEX; e.g. DECADRON®) and corticosteroids such as, for example, prednisone, nucleoside enzyme inhibitors such as, for example, hydroxyurea, amino acid depleting enzymes such as, for example, asparaginase, leucovorin, folinic acid, raltitrexed, and other folic acid derivatives, and similar, diverse antitumor agents. The following agents may also be used as additional agents: amifostine (e.g. ETHYOL®), dactinomycin, mechlorethamine (nitrogen mustard), streptozocin, cyclophosphamide, lomustine (CCNU), doxorubicin lipo (e.g. DOXIL®), gemcitabine (e.g. GEMZAR®), daunorubicin lipo (e.g. DAUNOXOME®), procarbazine, mitomycin, docetaxel (e.g. TAXOTERE®), aldesleukin, carboplatin, cladribine, camptothecin, 10-hydroxy 7-ethyl-camptothecin (SN38), floxuridine, fludarabine, ifosfamide, idarubicin, mesna, interferon alpha, interferon beta, mitoxantrone, topotecan, leuprolide, megestrol, melphalan, mercaptopurine, plicamycin, mitotane, pegaspargase, pentostatin, pipobroman, plicamycin, tamoxifen, teniposide, testolactone, thioguanine, thiotepa, uracil mustard, vinorelbine, or chlorambucil.

The use of the cytotoxic, chemotherapeutic and other anticancer agents described above in chemotherapeutic regimens is generally well characterized in the cancer therapy arts, and their use herein falls under the same considerations for monitoring tolerance and effectiveness and for controlling administration routes and dosages, with some adjustments. Typical dosages of an effective cytotoxic agent can be in the ranges recommended by the manufacturer, and where indicated by *in vitro* responses or responses in animal models, can be reduced by up to about one order of magnitude concentration or amount. Thus, the actual dosage will depend upon the judgment of the physician, the condition of the patient, and the effectiveness of the therapeutic method based on the *in vitro* responsiveness of the primary cultured malignant cells or histocultured tissue sample, or the responses observed in the appropriate animal models.

In a particular embodiment, the invention related to the method of treatment as described above further comprises administering at least one anti-neoplastic or anti-tumor drug.

In a particular embodiment, the present invention relates to a method of treatment comprising administering to a subject in need of treatment, an effective amount of:
- at least one polypeptide as described above, preferably P3 (SEQ ID NO:1) or P4 (SEQ ID NO: 2); and
- a platinum complex,
wherein preferably said effective amount is sufficient to inhibit cancer or tumor growth.

Said platinum complex may be preferably selected form the group consisting of cisplatin, carboplatin, oxaliplatin, satraplatin, picoplatin, nedaplatin, triplatin, lipoplatin and combination thereof.

Applicant surprisingly found that the administration of both a polypeptide consisting in SEQ ID NO: 1 and a platinum complex showed synergistic effect.

In one embodiment, said polypeptide and said platinum complex are administered simultaneously. In another embodiment, said polypeptide and said platinum complex are administered sequentially. Preferably, said polypeptide and said platinum complex are administered by separate routes.

In a particular embodiment of the method of treatment of the invention, the subject to be treated is tested or was previously tested for the presence of cancer cells expressing CD9P-1.

Said identification of cancer cells expressing CD9P-1 may be carried out by any method well known in the art, such as for example immunohistochemistry, PCR, hybridation in situ, using primers, sequences or antibodies specific for CD9P-1. Examples of antibodies anti- CD9P-1 are the following: SAB2700379, HPA017074 (Sigma).

An object of the invention is a diagnostic kit for selecting a subject in need for the treatment of the invention. In one embodiment, said diagnostic kit comprises immunoassay reagents or primers or sequences to measure the expression of CD9P-1. CD9P-1 is used as a biomarker for the companion diagnostic test.

Another object of the invention is a method for treating cancer in a subject in need thereof, comprising:
- assessing the presence of cancer cells expressing CD9P-1 in the subject,
- if cancer cells expressing CD9P-1 are detected, then treating the subject as described here above.

Another object of the invention is a method for treating cancer in a subject in need thereof, comprising:
- performing a companion diagnostic test of the subject before treatment, wherein said companion diagnostic test comprises detecting the presence of cancer cells expressing CD9P-1,
- according to a positive result of the companion diagnostic test, treating the subject as described here above.

The invention also relates to a method for treating cancer and/or tumor in a subject in need thereof wherein cancer cells and/or tumor cells express CD9P-1.

This invention will be better understood from the Experimental Details that follow. However, one skilled in the art will readily appreciate that the specific methods and results discussed are merely illustrative of the invention as described more fully in the claims which follow thereafter, and are not to be considered in any way as limited thereto.

### BRIEF DESCRIPTION OF THE DRAWINGS

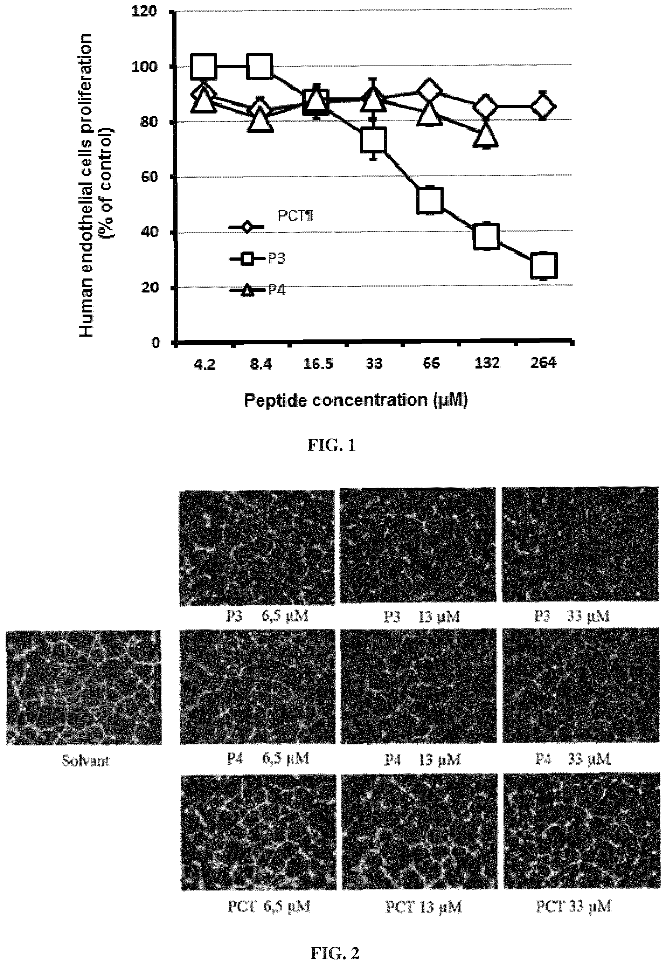

**Figure 1** is a graph showing the dose-dependent inhibition of HUVECs proliferation by peptides 3 and 4. Human endothelial cells proliferation is expressed in percentage of proliferation of a control consisting in HUVECs cultured in the presence of solvent; and is represented as a function of peptide concentration (in µM). Results obtained for peptides PCT (peptide control), P3 and P4 are compared.
**Figure 2** is a photograph showing the inhibition of peptides 3 and 4 over the formation of capillary networks by HUVECs cultured on a Matrigel® extracellular matrix. Capillary networks are evidenced by fluorescence labeling with calcein, with a microscope (zoom x40). Results are shown for peptide concentrations of 6.5µM, 13µM and 33µM. Solvent is used as a control.

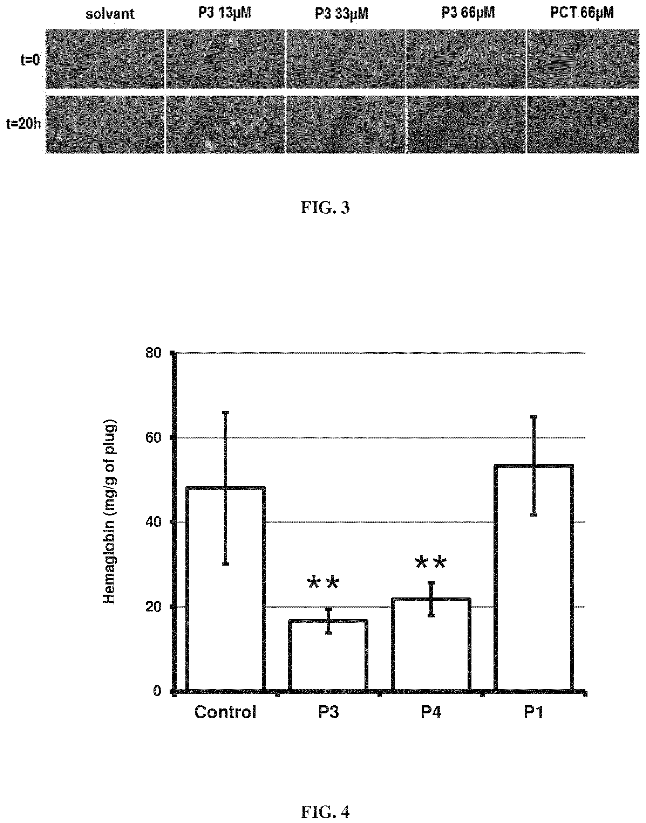

**Figure 3** is a photograph showing the inhibition of HUVECs migration by P3. Migration of HUVECs over a wound generated with a chisel tip is compared at t=0h and t=20h in presence of solvent (control), PCT (66µM) or P3 (13µM, 33µM and 66µM).
**Figure 4** is a histogram showing the level of hemoglobin measured in matrigel® implants, enriched in tumor cells and subcutaneously transplanted in Swiss-nude/nude mice treated for 12 days with intraperitoneal injections of peptides P3, P4, P1 (at 10mg/kg) or by solvent alone. **: p<0.01.

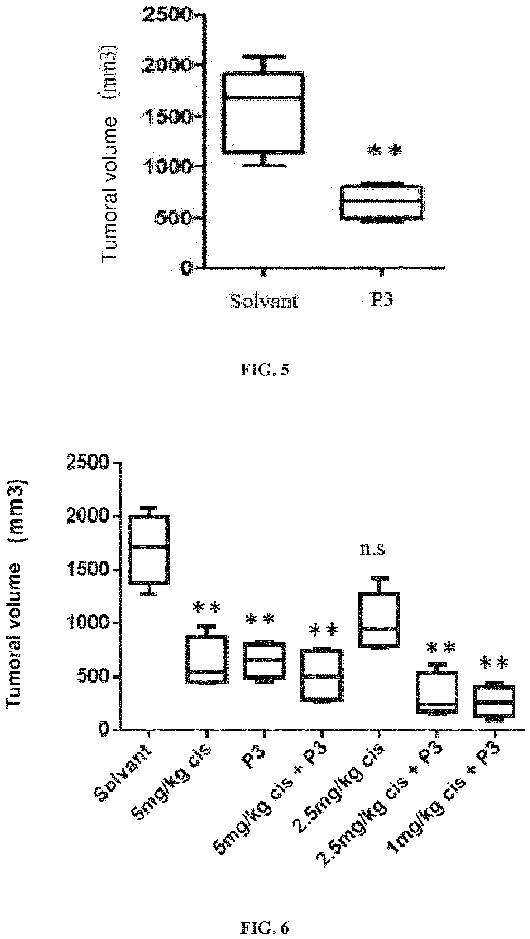

**Figure 5** is a histogram showing the effects of peptides Solvent and P3 in vivo on the tumoral growth of NCI-H460 cells grafted in Swiss-nude/nude mice. Tumoral volume (in mm3) is represented as a function of the peptide tested. Solvent is used as a control. **: p<0.01.
**Figure 6** is a histogram showing the *in vivo* inhibition of tumoral growth under the effect of P3 and/or of cisplatin. Tumoral volume (in mm³) at the end of the treatment (13 days) is represented as a function of the received treatment. PCT: control peptide; cis: cisplatin; n.s.: non significant.

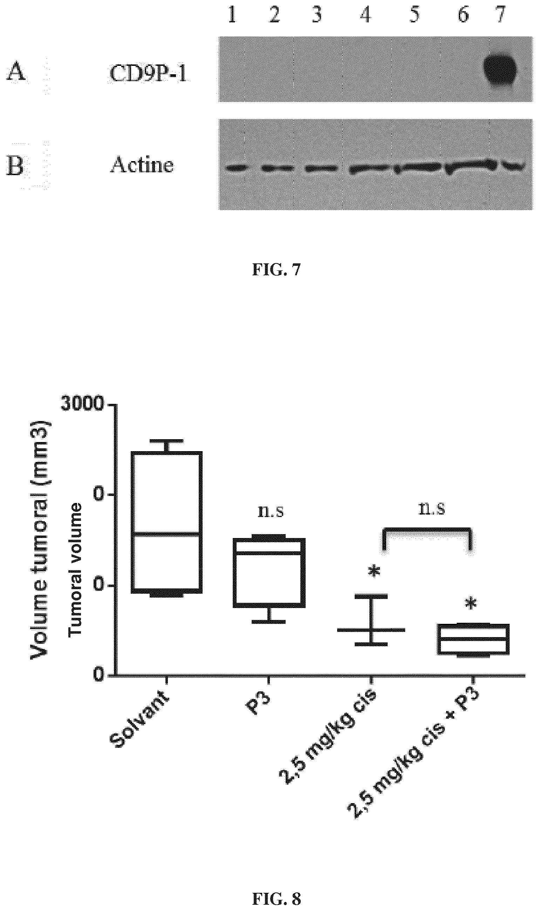

**Figure 7** is a photograph of Western Blots showing the expression of CD9P-1 over UMUC-3 tumoral cells lysates. Increasing quantities of proteins were loaded. Lane 1: 10µg; lane 2: 20µg; lane 3: 30µg; lane 4: 40µg; lane 5: 50µg; lane 6: 60µg; lane 7: 23µg of BT20 lysate (positive control). The Western Blot of line A has been performed with an anti-CD9P-1 monoclonal antibody. The Western Blot of line B has been performed with an anti-actin monoclonal antibody.
**Figure 8** is a histogram showing the effects of peptide P3 and cisplatin, administered in combination or alone, on the tumoral growth *in vivo* of UMUC-3 cells. The histogram shows the distribution of tumoral volume (in mm3) at the end of the treatment (13 days) as a function of the treatment received. Solvent was used as a control, n.s.: non significant; *: p<0.05.

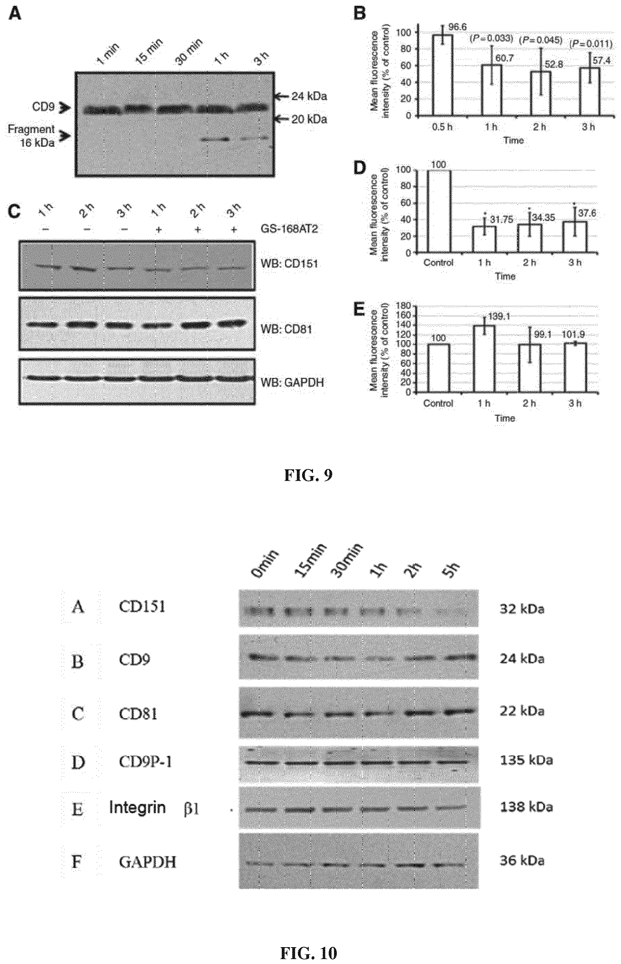

**Figure 9A** is a photograph showing the degradation of CD9 in HUVECs incubated with GS-168AT2. Columns correspond to Western Blot results obtained with anti-CD9 antibody after 1 min, 15 min, 30 min, 1h or 3h of incubation.
**Figure 9B** is a histogram showing the degradation of CD9 in HUVECs incubated with GS-168AT2. Bands corresponding to CD9 in Western Blot are quantified, normalized with respect to GAPDH and expressed in percentage with respect to HUVECs incubated with vehicle. Quantity of CD9 is represented as a function of the peptide tested.
**Figure 9C** is a photograph showing the degradation of CD151 in HUVECs incubated with either vehicle or GS-168AT2. Lines from top to bottom correspond to Western Blot results obtained with anti-CD151 antibody; with anti-CD81 antibody and anti-GAPDH antibody.
**Figure 9D** is a histogram showing the degradation of CD151 in HUVECs incubated with GS-168AT2. Bands corresponding to CD151 in Western Blot are quantified, normalized with respect to GAPDH and expressed in percentage with respect to HUVECs incubated with vehicle. Quantity of CD151 is represented as a function of the peptide tested.
**Figure 9E** is a histogram showing the degradation of CD81 in human EC incubated with GS-168AT2. Bands corresponding to CD81 in Western Blot are quantified, normalized with respect to GAPDH and expressed in percentage with respect to HUVECs incubated with vehicle. Quantity of CD81 is represented as a function of the peptide tested.
**Figure 10** is a photograph showing the *in vitro* effects of peptide P3 over the expression of CD151, CD9, CD81, CD9P-1 and integrin β1 chain in HUVECs. Lines A to E respectively correspond to Western blot results obtained after incubation with 33µM P3 for up to 5h incubation, with an anti-CD151 antibody (lane A); an anti-CD9 antibody (lane B); an anti-CD81 antibody (lane C); an anti-CD9P-1 antibody (lane D); an anti-β1 integrin antibody (lane E) and an anti-GAPDH antibody (lane F).

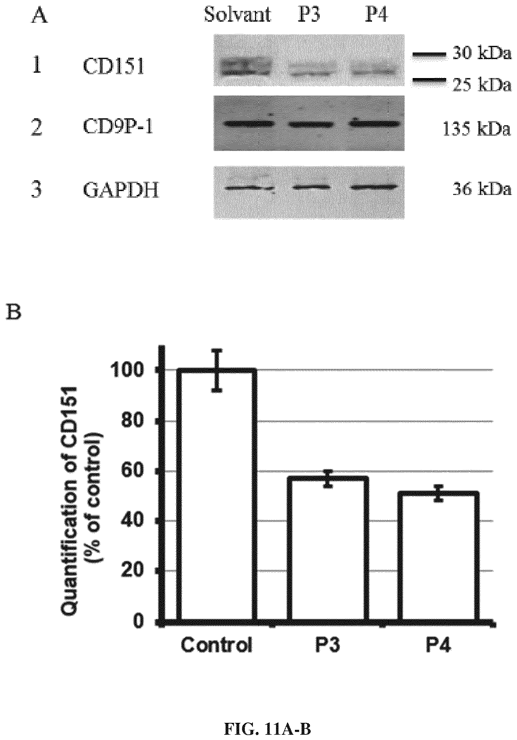

**Figure 11A** is a photograph showing the degradation of CD151 in presence of P3 and P4 in matrigel® implants enriched in NCI-H460 cells. Lines 1 to 3 correspond to Western Blot results obtained with anti-CD151 antibody (lane 1); with anti-CD9P-1 antibody (lane 2) and anti-GAPDH antibody (lane 3).
**Figure 11B** is a histogram showing the degradation of CD151 in presence of P3 and P4 in matrigel® implants enriched in NCI-H460 cells. CD151, corresponding to the band of 28KDa in Western Blot, is quantified, normalized with respect to GAPDH and expressed in percentage with respect to the solvent. Quantity of CD151 is represented as a function of the peptide tested.

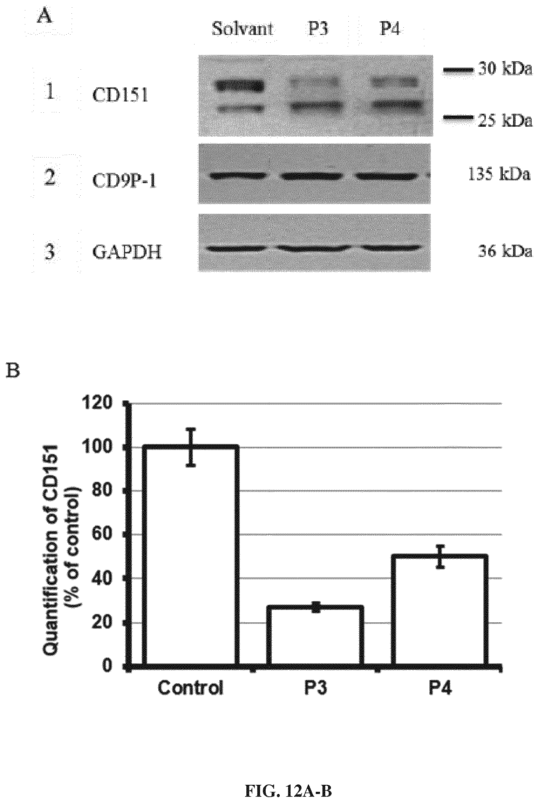

**Figure 12A** is a photograph showing the degradation of CD151 in xenografts of NCI-H460 cells when Swiss-nude/nude mice are treated with peptides 3 and 4. Lines 1 to 3 correspond to Western Blot results obtained with anti-CD 151 antibody (lane 1); with anti-CD9P-1 antibody (lane 2) and anti-GAPDH antibody (lane 3).
**Figure 12B** is a histogram showing the degradation of CD151 in xenografts ofNCI-H460 cells when Swiss-nude/nude mice are treated with peptides 3 and 4. Bands corresponding to CD151 in Western Blot are quantified, normalized with respect to GAPDH and expressed in percentage with respect to xenografts of mice treated with the solvent alone. Quantity of CD151 is represented as a function of the peptide tested.

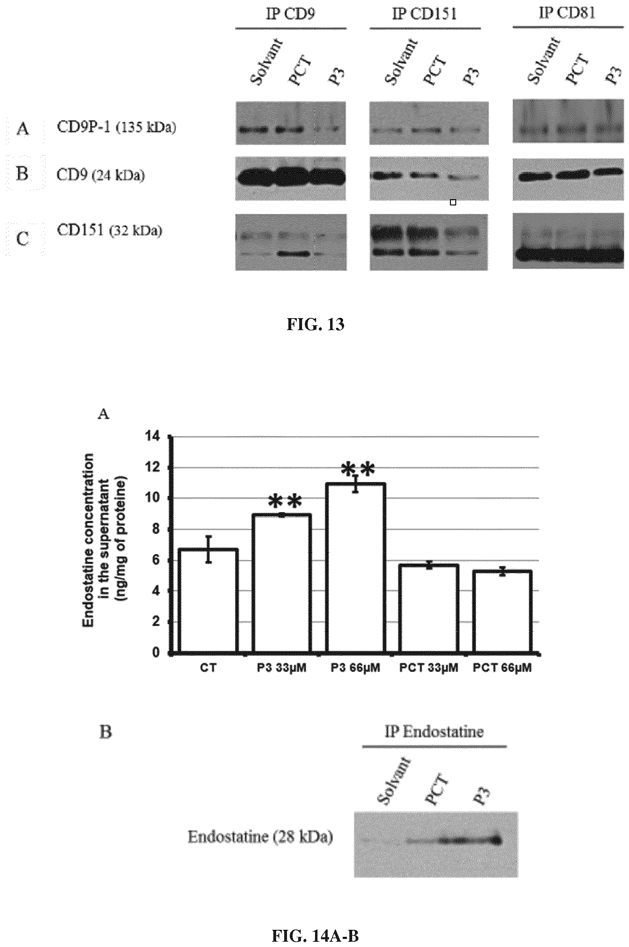

**Figure 13** is a photograph representing the dissociation of CD9-CD9P-1, CD9P-1-CD 151 and CD9-CD151 complexes by P3 in HUVECs. HUVECs incubated for 2h with 33µM P3, PCT or solvent in EGM2-MV medium were lysed in brij 97 buffer, then CD81, CD9 or CD151 were immunoprecipitated with the corresponding antibody. Line A: Western blot results obtained with anti-CD9P-1 antibody from immunoprecipitated CD81, CD9 and CD151. Line B: Western blot results obtained with anti-CD9 antibody from immunoprecipitated CD81, CD9 and CD151. Line C: Western blot results obtained with anti-CD151 antibody from immunoprecipitated CD81, CD9 and CD151. PCT: control peptide. IP: immunoprecipitation.
**Figure 14A** is a histogram showing the increase of endostatin production in HUVECs supernatants incubated with P3 *in vivo.* Endostatin concentration in supernatants, quantified by ELISA (expressed in ng/mg of proteins in the cell lysate) is represented as a function of P3 concentration. **: p<0.01 (non parametric student assay against the solvent).
**Figure 14B** is a photograph showing the increase of endostatin production in HUVECs supernatants incubated with P3 *in vivo.* Western Blot results were obtained after immunoprecipitation of endostatin in a same volume of supernatant by the anti-COL18A1 polyclonal antibody.

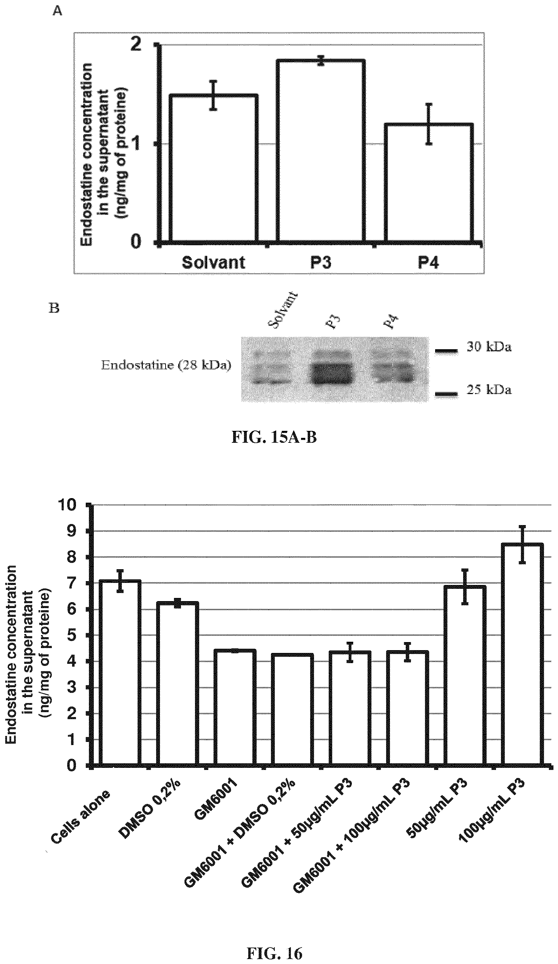

**Figure 15A** is a histogram showing the P3-induced increase of circulating endostatin in vivo. The histogram displays the endostatin concentration in the supernatant (in ng/mg of proteins) as a function of the peptide tested. Endostatin concentration was measured by ELISA on sera obtained in the *in vivo* study.
**Figure 15B** is a photograph showing the P3-induced increase of circulating endostatin *in vivo.* The image was obtained by Western Blot of sera obtained in the in vivo study with the anti-COL18A1 antibody.
**Figure 16** is a histogram showing the inhibition of endostatin production induced by P3 and/or by GM6001 in HUVECs *in vitro.* The endostatin concentration in the supernatant (in ng/mg of proteins) is represented as a function of the treatment provided to cells. Endostatin concentration in the supernatants was measured by ELISA (non parametric student assay against the solvent).

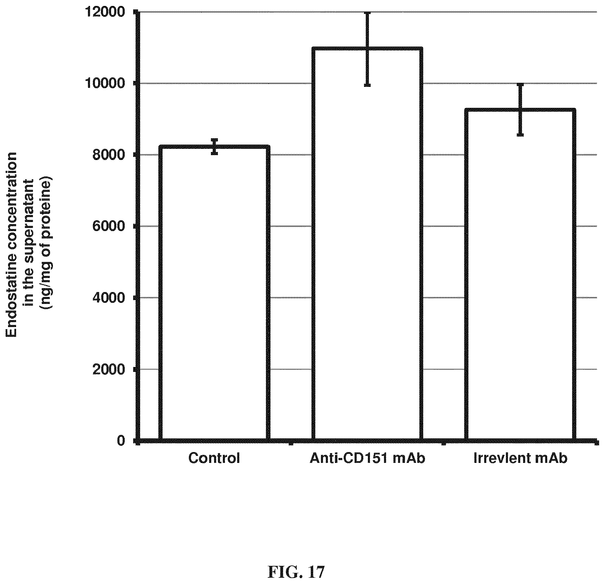

**Figure 17** is a histogram showing the in vivo production of endostatin in HUVEC supernatants in presence of an anti-CD151 antibody. The endostatin concentration in the supernatant (in ng/mg of proteins) is represented as a function of the treatment provided to cells. Endostatin concentration in the supernatants was measured by ELISA (non parametric student assay against the solvent).

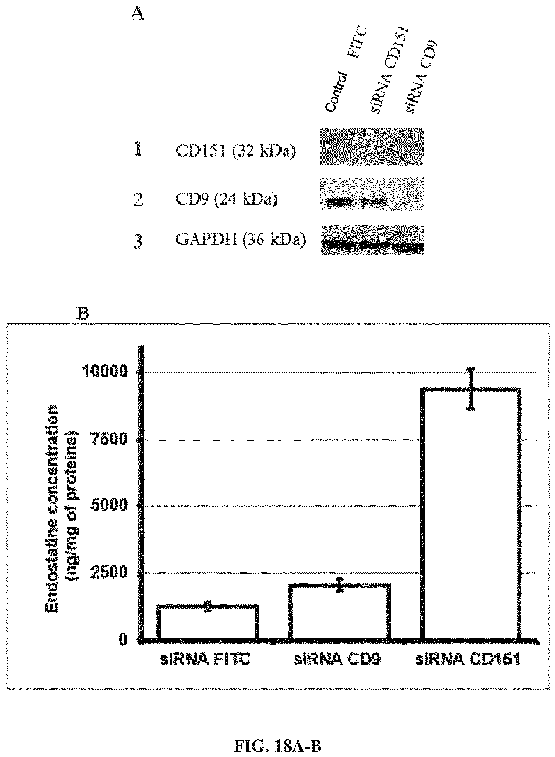

**Figure 18A** is a photograph showing that the increase of endostatin production is linked to the CD151 depletion in HUVECs. Images correspond to Western Blot results obtained with HUVEC lysates after treatment with various siRNAs (siRNA FITC, siRNA CD151 or siRNA CD9).
**Figure 18B** is a histogram showing that the increase of endostatin production is linked to the CD151 depletion in HUVECs. The endostatin concentration in the supernatant (in ng/mg of proteins) is represented as a function of the treatment provided to cells. Endostatin concentration in the supernatants was measured by ELISA in HUVEC supernatants incubated with siRNAs for 48h.

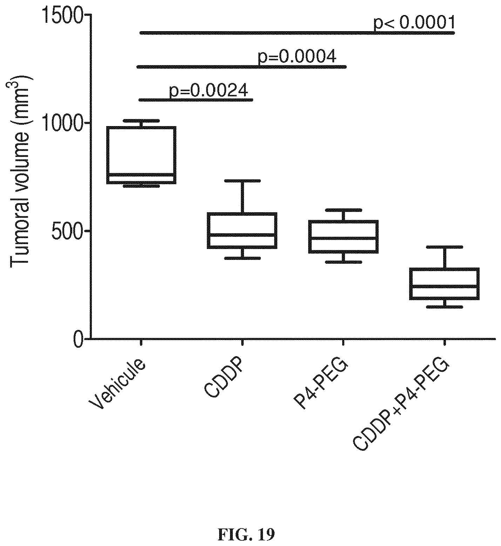

**Figure 19** is a histogram showing the effects of PEGylated peptide P4 and cisplatin, administered in combination or alone, on the tumoral growth *in vivo* of Calu-6 cells. The histogram shows the distribution of tumoral volume (in mm3) at the end of the treatment (16 days) as a function of the treatment received. Solvent was used as a control.

### EXAMPLES

### Material and methods

### Cell culture

Endothelial cells from human ombilical vein cord (HUVECs) were cultivated in an EGM2-MV media (media EBM2 containing 5% SVF; 0.4% human FGF-2 ; 0.1% VEGF; 0.1% IGF-1 with substition of Arginin for glutamin at position 3 (R3-IGF-1); 0.1% human EGF; 0.04% hydrocortisone; 0.1% ascorbic acid; 0.1% gentamicine-100.

NCI-H460 cells (human cancer cells from non-small cell lung) were cultivated in RPMI1640 media supplemented with 2.5 g/L glucose; 10 mM HEPES; ImM sodium pyruvate; 10% SVF.

UMUC-3 cells (human bladder cancer cells) were cultivated in a MEM NEAA media supplemented with 10 % SVF; 1 mM sodium pyruvate; and 2mM glutamin.

### Method to select the peptides

Peptides were synthesized by GENECUST and solubilized in 10% DMSO at 10 mg/ml for in vitro tests and 1 mg/ml for in vivo tests. The control peptide (PCT) (SEQ ID NO: 5: NQKGCYDLVT; molecular weight 1140 Da) was used at the same final concentration as the tested peptides.

### Cellular proliferation test

Evaluation of cellular proliferation was performed using 4,5-diMethylThiazol-2-yl)-2,5 diphenylTetrazolium bromide (MTT) test. After 18 hrs of culture, the media was changed by EBM2 supplemented with gentamycine and 5% SVF. Different concentrations of peptides were added under a volume of 10µL/well. Cells were incubated for 48 hrs at 37°C. MTT was added (50µL/well) to the cells for 3hrs.

### Capillaries formation evaluation

Evaluating the formation of capillaries on Matrigel® was performed by angiogenesis test in vitro described in Grant et al (1989 Cell 58, 933-943). Matrigel® was dropped down the wells of the 96 well plates and maintained for 30 min at 37°C for the Matrigel® solidification. HUVECs cells were then seeded and 5µL of peptide added.

Plates were then incubated for 18 hrs at 37°C cells were then washed with calcein solution (50µL/well at 8µM) and incubated for 20 min. HUVECs alive metabolize calcein in a green fluorescent component.

### Migration test following the wound test

The migration of the cells was evaluated according to the wound test (Sato and Rifkin 1988 J Cell Biol 107, 1199-1205). HUVECs were cultivated to confluence in an EGM2-MV media. The wound width was measured using the software "Analysis" (Soft Imaging System Gmbh Digital, Olympus). Peptides to be tested were then added to the media.

### Angiogenesis test in vivo

Implants of Matrigel® represent an *in vivo* model of angiogenesis. NCI-H460 cells are used to provide growth factors necessary for blood vessels formation within the Matrigel® matrix. The mixture was then injected sub-cutanously in the Swiis nu/nu mice. Daily treatments with the peptides (1 mg/mL) were performed by injections intraperitoneally. Implants were collected after 12 days.

### Hemoglobin levels quantifications within implants of Matrigel®

The dosage of hemoglobin in implants was used as an indicator of the vessels of the implant. The implants were weighted and crushed.

### Tumor growth test in vivo

Swiss nu/nu female mice aged 5-6 weeks were anesthetized with xylazine (12 mg/kg) and ketamine (80 mg/kg). Tumor cells (NCI-H460 or UMUC-3) were implanted sub-cutanously in the mice flanks. When the tumor reaches 100 mm³ after 10 days of inoculation, animals are randomized and treated by injection every other day with peptides, cisplatin intraperitoneally for 13 days. Tumor sizes were measured and tumor volume was calculated. Mice were weighted to detect symptoms of pain.

### Suppression of tetraspanins expression

HUVECs were transfected with siRNA using transfecting agent GENJET according to the manufacturer's instructions. After 48 hrs of incubation, supernatants were collected, protein expression tested by western blots and endostatin quantified.

| CD151 human pool Primers sc-42829 A, B and C (Santa Cruz Inc) | sequence | SEQ ID NO |
|---|---|---|
| Sens A | CGAGAAGAAGACAACAUGUtt | SEQ ID NO: 6 |
| Anti-sens A | ACAUGUUGUCUUCUUCUCGtt | SEQ ID NO: 7 |
| Sens B | CCUCCAACAUCUACAAGGUtt | SEQ ID NO: 8 |
| Anti-sens B | ACCUUGUAGAUGUUGGAGGtt | SEQ ID NO: 9 |
| Sens C | CAGGUCUUUGGCAUGAUCUtt | SEQ ID NO: 10 |
| Anti-sens C | AGAUCAUGCCAAAGACCUGtt | SEQ ID NO: 11 |

| CD9 human pool Primers sc-35032 A, B and C (Santa Cruz Inc) | sequence | SEQ ID NO |
|---|---|---|
| Sens A | CCAUCCACUAUGCGUUGAAtt | SEQ ID NO: 12 |
| Anti-sens A | UUCAACGCAUAGUGGAUGGtt | SEQ ID NO: 13 |
| Sens B | CUGUCCUGAUGCCAUCAAAtt | SEQ ID NO: 14 |
| Anti-sens B | UUUGAUGGCAUCAGGACAGtt | SEQ ID NO: 15 |
| Sens C | GGCAUGAUCUUCAGUAUGAtt | SEQ ID NO: 16 |
| Anti-sens C | UCAUACUGAAGAUCAUGCCtt | SEQ ID NO: 17 |

The present invention is further illustrated by the following examples.

### Example 1: Dose-dependent inhibition of HUVECs proliferation by P3 and P4

Cells were grown for 48h in EGM2-MV and proliferation was quantified by MTT assay. Results are displayed in Fig. 1 and are expressed as a percentage of cell proliferation with respect to HUVECs cultured in the presence of solvent.

The effect of 3 peptides was tested on the HUVECs proliferation. No effect was observed with P1 (SEQ ID NO: 4: VSWFAVHSFGLDKAPVLLSS) on the proliferation (results not shown), as for the PCT (SEQ ID NO: 5: NQKGCYDLVT).

On the contrary, peptide 3 (P3 - SEQ ID NO: 1) inhibits HUVECs proliferation in a dose-dependent manner (**Fig 1**). At 66 µM, 50% ± 1.2% (p<0.001; n=3) of the HUVECs proliferation is inhibited by P3. At 264 µM, proliferation in P3 condition, is 27.3% ± 0.3% (p<0.001 relating to PCT; n=3). The tests performed with peptide 4 (P4 - SEQ ID NO: 2) showed 20% inhibition on the proliferation of HUVECs until 66 µM of concentration (**Fig 1**). At a concentration of 132 µM, P4 enhances inhibition on HUVECs proliferation of 26% ± 1.75% (p=0.0121 relating to PCT; n=3).

### Example 2: P3 and P4 inhibition of the formation of capillary networks by HUVECs cultivated on Matrigel®

Peptides were added to the culture medium (EGM2-MV) at concentrations 6.5µM, 13µM and 33µM after inoculation of cells. Plates were incubated at 37°C for 18h. Fluorescence labeling was achieved by addition of calcein. Pictures were shot with a fluorescence microscope equipped with a camera (zoom x40). Results are displayed in figure 2.

The formation of network structures observed is spontaneous in presence of solvent. The addition of PCT has no effect on the capillary tubes formation by HUVECs (**Fig. 2**). On the opposite, P3 enhances dose-dependent decreases of both capillaries length and junctions number, illustrating a significant *in vitro* anti-angiogenic activity (**Fig. 2**). P4 also demonstrates significant decrease of both capillaries length and junctions number illustrating a significant *in vitro* anti-angiogenic activity.

### Example 3: P3-dependent migration inhibition of HUVECs

A wound has been performed with a chisel tip on the cell layer. The HUVECs migration following the addition of peptides was evaluated by the wound assay and the progress of the migration front of the HUVECs was analyzed after 20hrs of culture. Results show that the wounds to which the solvent or the PCT was added are completely closed following 20hrs of incubation. Pictures were shot with a microscope equipped with a camera (zoom x40). Cells incubated with 13µM of P3, a partial inhibition of the scar is observed. At 33µM, P3 seems to prevent completely HUVECs from migration and progress of migration front is significantly inhibited or almost stopped (**Fig 3**).

### Example 4: Effect of P3 and P4 on tumoral growth in vivo

An *in vivo* model of angiogenesis as described in Passaniti et al. (1992 Experimental gerontology 27, 559-566) was utilized to evaluate the effect of P3 and P4. Briefly, cancer cells were incorporated into Matrigel®, cancer cells provide growth and angiogenic factors necessary for blood vessel formation. The mixture was injected immediately under a sub-cutaneous form of the mice flanks. The day following the inoculation, 20 mice (Swiss nu-nu) were randomized in 4 groups containing 5 mice each and the treatments started as follows: mice of group 1 were treated by 200µL/injection/day of solvent alone for 12 successive days; mice of group 2 were treated by 200µL/injection of a P1 solution (1mg/mL) in a solvent/injection/day for 12 successive days; mice of group 3 were treated by 200µL of a P3 solution (1mg/mL) in a solvent/injection/day for 12 successive days; mice of group 4 were treated by 200µL of a P4 solution (1mg/mL) in a solvent/injection/day for 12 successive days.

Mice were sacrificed at day 13 and the implants were collected and examined. Results show that the implants of group 1 (control mice having the solvent alone) and group 2 (mice having 10 mg/kg of P1) are characterized by a red color while implants collected from group 3 (mice having 10 mg/kg of P3) and group 4 (mice having 10 mg/kg of P4) are characterized by a yellow color with some red points, suggesting an inhibition of *in vivo* angiogenesis. The dosage of hemoglobin in the implants was used to quantify the blood and hence evaluating the density of functional vessels within the implants. The analysis of the implants treated by P3 and P4 shows that these peptides induce an inhibition of hemoglobin amount of 67.2 ± 6.5% (p=0.0012) and 29.2 ± 8.4% (p=0.0029) compared to group 1 (**Fig 4**). These results demonstrate that P3 and P4 present anti-angiogenic properties.

### Example 5: Effect of P3 on tumoral growth - NCI-H460 model

Evaluating the effect of the peptides on tumor growth *in vivo* was performed. Cancer cells NCI-H460 were injected by sub-cutaneous administration in the Swiss-nu/nu mice flanks (n=20). 10 days following inoculation of tumor cells, mice were randomized in 2 groups of 5 mice. Mice of different groups were treated as follow: mice of group 1 were treated with solvent (200µL/injection/48hrs); mice of group 2 were treated with P3 (200µL of 1 mg/mL of P3 in the solvent/injection/48hrs). All the treatments were performed by intraperitoneal injection for 13 successive days.

After 13 days of treatment, results show that the average of tumoral volume of treated tumors with the solvent is 1560 ± 188.3 mm³ (n=5) (p=0.2625; n=5) (**Fig 5**). On the contrary, the average of tumoral volume of mice treated with P3 is 651.5 ± 80.97 mm³ (p=0.005; n=5) (**Fig 5**), indicating an inhibition of the tumor growth *in vivo* of 64.79 ± 4.37% (p<0.05).

In parallel, to investigate the toxic effect of peptides tested on mice, the mice weights were measured along the treatment. No significant changes of weights were noticed as shown in Table 1.

**Table 1: Average weights of mice at the beginning of the treatment (day 0) and at the end (day 13), and % of weights differences of the mice during the treatment by Solvent and P3.**

| **Treatment** | **Weighted average of mice at the beginning of the treatment (g)** | **Weighted average of mice after 13 days of the treatment (g)** | **Weight difference between day 1 and day 13 (%)** |
|---|---|---|---|
| Solvent | 22.9 ± 0.3 % | 24.8 ± 0.7 % | 7.7 ± 3% |
| P3 | 22.1 ± 2.3 % | 24.6 ± 0.4 % | 10.2 ± 9% |

### Example 6: Combined effect of P3 and cisplatin effect on tumor growth

To compare the biological activity of P3 with GS-168AT2, a study on the anti-tumoral activity of P3 alone or in combination with cisplatin *in vivo* was performed.

The inoculation of NCI-H460 cells (tumoral volume of 100 to 150 mm³), mice (n=35) were randomized in 7 groups comprising 5 mice each. Mice from the different groups were treated as follow: group 1with the solvent (200 µL/injection/48hrs); mice of group 2 were treated with cisplatin (5mg/kg). Mice of group 3 were treated with P3 (10 mg/kg); mice of group 4 were treated alternately with P3 (10 mg/kg) and cisplatin (5 mg/kg), mice of group 5 were treated with cisplatin (2.5 mg/kg); mice of group 6 were treated alternately with P3 (10 mg/kg) and cisplatin (2.5 mg/kg); mice of group 7 were treated alternately with P3 (10 mg/kg) and cisplatin (1 mg/kg). All the treatments were performed by intraperitoneal injection for 13 successive days.

Results indicate that monotherapy by P3 (10 mg/kg) shows a therapeutic efficacy close or similar to the one of cisplatin (5 mg/kg). The average tumoral volume of the group treated with P3 is about 651.5 ± 80.9 mm³ (p=0.0013; n=5) while the average tumoral volume of the control group is 1698 ± 164.9 mm³ (n=5), indicating that the treatment with P3 alone induces inhibition of tumor growth *in vivo* of 61.61 ± 4.74 % (p<0.05; n=5) (**Fig 6**). The average tumoral volume of mice treated with cisplatin alone (5 mg/kg) is 626 ± 118.5 mm³ (p=0.0019; n=5) indicating that this treatment induces inhibition of tumor growth similar to the effect of P3 (**Fig 6**). The effect of bi-therapy alternately daily the cisplatin (5 mg/kg) and P3 (10 mg/kg) shows that the average tumoral volume of the group treated with this bi-therapy is 551.9 ± 122.4 mm³ (p=0.0012; n=5). Consequently, this bi-therapy inhibits the tumor growth *in vivo* by 69.85 ± 7.2% (p<0.05; n=5).

Decrease of doses of cisplatin (2.5 then 1 mg/kg) administered in monotherapy or bi-therapy in alternately daily with P3 (10 mg/kg). The aim is to maintain the therapeutic efficacy while limiting the potential systemic effects observed with cisplatin at a 5 mg/kg dose or eventual resistance mechanisms depending on doses of chemotherapeutic agents. The half dose reduction of cisplatin (2.5 mg/kg) enhance a decrease in the anti-tumoral efficacy (average tumoral volume is 1017 ± 119.8 mm³; p=0.011; n=5). However, the treatments by bi-therapy alternating daily with cisplatin (2.5 mg/kg) and P3 (10 mg/kg) or cisplatin (1 mg/kg) and P3 (10 mg/kg) maintain the therapeutic efficacy with tumoral volume averages respectively of 318.7 ± 103.3 mm³; (p=0.0004; n=5) and of 265.4 ± 72.1 mm³ (p=0.0002; n=5) (**Fig 6**).

Furthermore, mice treated with cisplatin (5 mg/kg) exhibit a significant loss of weight of 20 ± 7%; p=0.0159 comparing to the control; n=5) or in alternately with P3 (10 mg/kg) of 18 ± 11%; (p=0.079 comparing to the control; n=5). The decrease of the cisplatin dose by half (2.5 mg/kg) reduces the weight loss at 6 ± 3%. Changes in weight of mice treated alternately with cisplatin (2.5 mg/kg) and P3 (10 mg/kg) is of -7 ± 1%. The reduction of the dose of cisplatin (1 mg/kg) does not enhance any weight changes as described in Table 2.

**Table 2: Average weights of mice at the beginning of the treatment (day 0) and at the end (day 13), and % of weights differences of the mice during the treatment by P3 and/ or cisplatin.**

| **Treatment** | **Weighted average of mice at the beginning of the treatment (g)** | **Weighted average of mice after 13 days of the treatment (g)** | **Weight difference between day 1 and day 13 (%)** |
|---|---|---|---|
| Solvent | 23.6 ± 1.2 % | 24.9 ± 1.3 % | 5 ± 2% |
| 5 mg/kg cis | 24.6 ± 1.6 % | 20.5 ± 1.2 % | -20 ± 7% |
| P3 | 24.6 ± 2.8 % | 26.2 ± 2.9 % | 6 ± 2% |
| 5 mg/kg cis + P3 | 23.7 ± 2.3 % | 20.2 ± 2.6 % | -18 ± 11% |
| 2.5 mg/kg cis | 24.8 ± 1.2 % | 23.5 ± 0.7 % | -6 ± 3% |
| 2.5 mg/kg cis + P3 | 25.5 ± 0.7 % | 23.8 ± 0.9 % | -7 ± 1% |
| 1 mg/kg cis + P3 | 24.5 ± 0.9 % | 25.9 ± 0.9 % | 5 ± 6% |

### Example 7: Effect of P3 on tumoral growth - UMUC model - demonstrating CD9P-1 dependency

P3 is derived from CD9P-1. To check if P3 interact with CD9P-1, P3 was tested on a tumor cell line that does not express CD9P-1. UMUC-3 cell line (a bladder tumor cell line) was identified in a screening assay as a cell line that does not express CD9P-1. Increasing amounts of lysates of UMUC-3 (10 to 60 µg) analyzed by western blots show that these cells do not CD9P-1 (**Fig 7A**).

The effect of P3 was then tested on the tumor growth in an *in vivo* model of xenograft of UMUC-3. UMUC-3 cells were inoculated for 10 days (tumor volume from 100 to 150 mm³, mice (n=20) were randomized in 4 groups containing 5 mice each. The different groups were treated as follow: mice of group 1 were treated with the solvent (200µL/injection/48hrs); mice of group 2 were treated with cisplatin (2.5 mg/kg); mice of group 3 were treated with P3 (10 mg/kg); mice of group 4 were treated alternately with cisplatin (2.5 mg/kg) and P3 (10 mg/kg). The tumoral volume of mice treated with P3 did not change significantly compared to the tumoral volume of mice treated with the solvent (**Fig 8**) which indicates that P3 does not affect the tumor growth of tumors that do not express CD9P-1. However, these tumors are reduced under cisplatin (2.5 mg/kg) treatment (**Fig 8**).

### Example 8: Effect of P3 on tetraspanins

The state of the art already demonstrated that GS-168AT2 is associated to CD9, CD151 and more weakly to CD81. The status of CD9, CD151 and CD81 were then assessed over time in presence of GS-168AT2 (Colin et al. 2011 Br J Cancer 105,1002-1011). HUVECs were incubated with GS-168AT2 (40 µg/mL), then lysed, and cell lysates were western blotted with anti-CD9, anti-CD151 and anti-CD81 antibodies. Results indicated that GS-168AT2 induces degradation of CD9 and CD151 after 1 hour of treatment (**Fig. 9A** and **C**). To investigate further, HUVECs incubated with GS-168AT2 were also analyzed by FACS. Results show important decreases in the amounts of CD9 and CD151 at the cell surface with time in the presence of GS-168AT2 relative to control, respectively about 40% less (P<0.05; **Fig. 9B**) and about 70% less (P<0.01; **Fig. 9D**). However, no significant changes of CD81 status following cell exposure to GS-168AT2 were observed (**Fig. 9C** and **E**). The degradation of CD9 and CD151 decreases the interactions between CD9 and CD181, CD9 and CD151 and CD81 and CD9P-1. Similar mechanisms were then studied with P3 on different cellular models *in vitro* and *in vivo.*

The *in vitro* effect of P3 on the tetraspanins (CD9, CD81 and CD151) and integrin β1 was performed on endothelial cells. HUVECs were incubated with P3 (33µM) at different time points. P3 induces a significant decrease of CD151 from 2hrs of incubation (**Fig 10A**)**.** However, neither CD9 expression (**Fig 10B**), CD81 (**Fig 10C**), and CD9P-1 (**Fig 10D**) nor integrin β1 are affected by P3.

Analysis of the expression of CD151 and CD9P-1 were performed on western blots on homogenates of implants of Matrigel® enriched with NCI-H460. CD151 profile is characterized by 2 bands on western blots; one at 28 kDa corresponds to CD151 and another one at 26 kDa corresponding to a degraded product of CD151. Results show a significant decrease of CD151 in lysates of mice treated by P3 and P4 (10 mg/kg) for 12 days (**Fig 11A-1**). However, CD9P-1 level does not change (**Fig 11A-2**). Quantification of the band corresponding to CD151 (**Fig 11B**) shows a decrease of 57 and 51% of the band corresponding to CD151 in lysates of mice treated with P3 and P4.

In lysates from xenografts NCI-H460 cells from mice treated for 13 days every other day by P3 or P4 (10 mg/kg), a decrease of CD151 level is observed respectively of 73% and 50% compared to mice treated with the solvent (**Fig 12A** and **B**). No change in CD9P-1 was observed (**Fig 12A**).

To study more precisely the effect of P3 on the associations of tetraspanins CD9, CD81, and CD151 between them and with CD9P-1, immunoprecipitation (IP) experiments were performed. In the control experiment (with the solvent), CD9P-1 co-precipitated with CD9 and CD81. A weak co-precipitation was observed between P3 and CD151. The associations between tetraspanins and CD9P-1 are not modified under PCT condition. However, the interactions between CD9P-1 and CD9 and between CD9P-1 and CD151 are decreased while the HUVECs are incubated for 2hrs with 33µM of P3. The association between CD9P-1 and CD81 is not modified by P3 (**Fig 13A**).

In the control experiment (with the solvent), the associations between CD9 and CD151 and between CD9 and CD81 are similar. Addition of P3 induces a decrease in the association between CD9 and CD151 but not between CD9 and CD81 (**Fig 13B**). Addition of P3 induces dissociation of the complex CD151-CD9 but not the complex CD151-CD81 (**Fig 13C**).

### Example 9: Effect of P3 on endostatin production in an in vitro and in vivo model

Endostatin is a fragment of collagen XVIII (O'Reilly et al. 1997 Cell 88, 277-285) and a renowned inhibitor of angiogenesis. Moreover, GS-168AT2 induces production of endostatin *in vivo* and *in vitro,* so the question relating to the capacity of P3 derived from GS-168AT2 to affect this activity is asked.

Endostatin levels were quantified by ELISAs on lysates of HUVECs incubated at rising concentrations of P3 or PCT for 48hrs. While PCT does not modify the level of endostatin compared to the solvent condition, an increase of endostatin production of 33 and 62% is observed under 33 and 66 µM of P3 respectively (**Fig 14A**).

Endostatin being issued from collagen XVIII, the antibody could detect both endostatin and collagen XVIII. An IP was then performed to confirm that the dosage by ELISA was due to endostatin (**Fig 14B**).

To study the effect of P3 on endostatin production *in vivo,* sera from xenograft mice with tumor cells NCI-H460 were analyzed by ELISA and western blots. An increase of 24% is observed in sera of mice treated by P3 (10mg/kg) compared to mice treated with the solvent (**Fig 15A**). Several forms of endostatin are observed by western blots. Analysis of sera by western blots confirms the increase of endostatin levels in the sera of mice treated with P3 and having a size of about 28 kDa (**Fig 15B**).

In order to evaluate the role of metalloproteinases (MMPs) in endostatin production, a polyvalent inhibitor of MMPs, GM6001 was used. The concentration of endostatin in supernatants is at 6.2 ng/mg of protein of the lysates/48hrs. A significant decrease of about 30% (4.4 ng/mg of protein of the lysates/48hrs) of endostatin was observed with the inhibitor of MMPs compared to the control. An increase of 10% (6.85 ng/mg of protein of the lysates/48hrs) of endostatin level with 33 µM of P3 and 46% (8.48 ng/mg of protein of the lysates/48hrs) with 66 µM of P3 was observed. Adding an inhibitor of MMPs 45 minutes before P3 addition, a significant decrease in endostatin levels of 30% compared to the control was observed (**Fig 16**). GM6001 inhibits endostatin production induced by P3. MMPs might then be implicated in endostatin production induced by P3.

Preceding results showed that a decrease of CD151 *in vitro* and *in vivo* and an increase of endostatin *in vitro* and *in vivo* were induced by P3. This raised the question about the link between endostatin production and decrease of CD151. Therefore CD151 was depleted and the endostatin levels were determined in supernatants. To do so CD151 expression was suppressed using anti-CD151 antibody and small interfering RNAs (pRNAi or siRNA) directed specifically against mRNA coding CD151.

HUVECs were incubated for 48hrs in an EGM2-MV media with an anti-CD 151 antibody (11G5a) or an irrelevant antibody (negative control unable to recognize any protein). Then endostatin levels were measured by ELISA in supernatants. An increase of 33% was observed when HUVECs were incubated with the anti-CD151 antibody (11G5a) compared to the basal production of endostatin where HUVECs cells are cultivated alone (**Fig 17**).

HUVECs cells were transfected with specific siRNA of CD151 and CD9. Protein expression was analyzed in western blots. In parallel, endostatin levels were measured by ELISA in supernatants. HUVECs transfected with siRNA specific of CD151 and CD9 suppressed their respective expression (**Fig 18A**). Levels of endostatin in supernatant of HUVECs cells transfected with siRNA were measured. No changes were observed between the control and the CD9 suppression of expression (1.26 ng/mg of protein lysates/48hrs and 2 ng/mg of protein lysates/48hrs respectively). On the contrary, levels of endostatin increased at 9.4 ng/mg of protein lysates/48hrs in HUVECs depleted in CD151 (**Fig 18B**). CD151 is thus involved in the production of endostatin.

### Example 10: Effect of PEGylated Peptide 4 on tumoral volume

All experiments were reviewed by Genopole's institutional animal care and use committee, and performed in accordance with institutional guidelines for animal care. Female BALB/c nu/nu mice (n=24) were used at 5-6 weeks of age. The animals were housed in laminar air-flow cabinets under pathogen-free conditions with a 14hrs light/10hrs dark schedule, and fed autoclaved standard chow and water ad libitum. Calu-6 cells (5x10⁶ cells in 200 µl of serum-free RPMI) were subcutaneously injected into the right flank of mice. After engraftment (10 days), tumor volume (TV) was measured (Balsari et al, Eur J Cancer. 2004 May;40(8):1275-81) and animals were randomized and separated into four groups of six animals each, to be treated by i.p. injection (200µl per injection) every other day for 16 days (eight injections). Control mice (group 1) received the vehicle (0.9% saline). Cis-diammine platinum II dichloride (CDDP; Sigma) was dissolved in 0.9% saline at 0.5mg/ml and injected at a dose of 5mg/kg (group 2). Pegylated peptide 4 (P4-PEG) was dissolved in vehicle at 1 mg/ml and injected at a dose of 10 mg/kg (group 3). In group 4, mice received both CDDP and P4-PEG. Tumor volume and body weight were measured every other day over the treatment period (16 days).

A significant decrease in tumor volume is observed in mice treated with CDDP alone, P4-PEG alone and both CDDP and P4-PEG. Consequently, P4-PEG has a significant anti-tumor effect.

## Claims

1. A polypeptide comprising at least one peptide selected from the group consisting in:
(i) a peptide of sequence GNYYCSVTPWVKS (SEQ ID NO: 1);
(ii) a peptide of sequence IHSKPVFITVKMDVLNA (SEQ ID NO: 2); and
(iii) a functional fragment or a variant or derivative thereof;
wherein said polypeptide comprises less than 50, preferably less than 40, preferably less than 30, preferably less than 20 contiguous amino acids of a sequence of SEQ ID NO: 3.

2. A polypeptide according to claim **1**, wherein said polypeptide is modified by the addition of one or more functional group such as a phosphate, acetate, lipid or carbohydrate group, and/or by the addition of one or more protecting group.

3. A polypeptide according to any one of claims **1** to **2**, wherein said polypeptide possesses anti-angiogenic and/or anti-tumoral activity.

4. A polynucleotide encoding a polypeptide according to any one of claims **1** to **3**.

5. A pharmaceutical composition comprising a polypeptide according to any one of claims **1** to **3** or a polynucleotide according to claim **4** and at least one pharmaceutically acceptable excipient.

6. A pharmaceutical composition according to claim **5**, further comprising at least one antiangiogenic, cytotoxic, chemotherapeutic or anti-cancer agent.

7. A pharmaceutical composition according to any one of claims **5** to **6**, further comprising a platinum complex selected from the group consisting of cisplatin and/or carboplatin.

8. A pharmaceutical composition according to any one of claims **5** to **7** comprising:
- a polypeptide according to claim **1**, wherein said polypeptide comprises a peptide of SEQ ID NO: 1, and a peptide of SEQ ID NO: 2; or
- a first polypeptide according to claim **1**, wherein said polypeptide comprises a peptide of SEQ ID NO: 1, and a second polypeptide according to claim **1**, wherein said polypeptide comprises a peptide of SEQ ID NO: 2.

9. The pharmaceutical composition according to any one of claims **5** to **8**, wherein said composition is in the form suitable for topical, systemic, oral, subcutaneous, transdermal, intramuscular, intravenous or intra-peritoneal administration.

10. A polypeptide according to any one of claims **1** to **3** or a pharmaceutical composition according to any one of claims **5** to **9** for use in the treatment of an angiogenesis-related disease.

11. A polypeptide according to any one of claims **1** to **3** or a pharmaceutical composition according to any of claims **5** to **9** for use in the treatment of cancer and/or of tumors in the human or animal body.

12. A polypeptide according to any one of claims **1** to **3** or a pharmaceutical composition according to any of claims **5** to **9** for use in the treatment of a cancer and/or tumor wherein cancer cells express CD9P-1.

13. A polypeptide or pharmaceutical composition for use in the treatment of a cancer and/or tumor in a subject in need thereof according to claim **12**, wherein prior to the treatment, the expression of CD9-P1 is tested in a sample comprising cancer and/or tumor cells of the subject.


SEQUENCE LISTING

<110> GENE SIGNAL INTERNATIONAL SA

<120> POLYPEPTIDES FOR TREATING CANCER

<130> CV - 341/EP/3

<160> 17

<170> BiSSAP 1.3

<210> 1
<211> 13
<212> PRT
<213> Artificial Sequence

<220>
<223> Peptide 3

<400> 1
Gly Asn Tyr Tyr Cys Ser Val Thr Pro Trp Val Lys Ser
1               5                   10

<210> 2
<211> 17
<212> PRT
<213> Artificial Sequence

<220>
<223> Peptide 4

<400> 2
Ile His Ser Lys Pro Val Phe Ile Thr Val Lys Met Asp Val Leu Asn
1               5                   10                  15
Ala

<210> 3
<211> 108
<212> PRT
<213> Artificial Sequence

<220>
<223> peptide 168A-T2

<400> 3
Met Ala Phe Asp Val Ser Trp Phe Ala Val His Ser Phe Gly Leu Asp
1               5                   10                  15
Lys Ala Pro Val Leu Leu Ser Ser Leu Asp Arg Lys Gly Ile Val Thr
            20                  25                  30
Thr Ser Arg Arg Asp Trp Lys Ser Asp Leu Ser Leu Glu Arg Val Ser
        35                  40                  45
Val Leu Glu Phe Leu Leu Gln Val His Gly Ser Glu Asp Gln Asp Phe
    50                  55                  60
Gly Asn Tyr Tyr Cys Ser Val Thr Pro Trp Val Lys Ser Pro Thr Gly
65                  70                  75                  80
Ser Trp Gln Lys Glu Ala Glu Ile His Ser Lys Pro Val Phe Ile Thr
                85                  90                  95
Val Lys Met Asp Val Leu Asn Ala Phe Lys Tyr Pro
            100                 105

<210> 4

<211> 20
<212> PRT
<213> Artificial Sequence

<220>
<223> Peptide 1

<400> 4
Val Ser Trp Phe Ala Val His Ser Phe Gly Leu Asp Lys Ala Pro Val
1               5                   10                  15
Leu Leu Ser Ser
            20

<210> 5
<211> 10
<212> PRT
<213> Artificial Sequence

<220>
<223> control peptide

<400> 5
Asn Gln Lys Gly Cys Tyr Asp Leu Val Thr
1               5                   10

<210> 6
<211> 21
<212> RNA
<213> Artificial Sequence

<220>
<223> siRNA CD151 sens A

<400> 6
cgagaagaag acaacaugut t                                                21

<210> 7
<211> 21
<212> RNA
<213> Artificial Sequence

<220>
<223> siRNA CD151 antisens A

<400> 7
acauguuguc uucuucucgt t                                                21

<210> 8
<211> 21
<212> RNA
<213> Artificial Sequence

<220>
<223> siRNA CD151 sens B

<400> 8
ccuccaacau cuacaaggut t                                                21

<210> 9
<211> 21

<212> RNA
<213> Artificial Sequence

<220>
<223> siRNA CD151 antisens B

<400> 9
accuuguaga uguuggaggt t                                                21


<210> 10
<211> 21
<212> RNA
<213> Artificial Sequence

<220>
<223> siRNA CD151 sens C

<400> 10
caggucuuug gcaugaucut t                                                21


<210> 11
<211> 21
<212> RNA
<213> Artificial Sequence

<220>
<223> siRNA CD151 antisens C

<400> 11
agaucaugcc aaagaccugt t                                                21


<210> 12
<211> 21
<212> RNA
<213> Artificial Sequence

<220>
<223> siRNA CD9 sens A

<400> 12
ccauccacua ugcguugaat t                                                21


<210> 13
<211> 21
<212> RNA
<213> Artificial Sequence

<220>
<223> siRNA CD9 antisens A

<400> 13
uucaacgcau aguggauggt t                                                21


<210> 14
<211> 21
<212> RNA
<213> Artificial Sequence

<220>
<223> siRNA CD9 sens B

<400> 14
cuguccugau gccaucaaat t                                                21


<210> 15
<211> 21
<212> RNA
<213> Artificial Sequence

<220>
<223> siRNA CD9 antisens B

<400> 15
uuugauggca ucaggacagt t                                                21


<210> 16
<211> 21
<212> RNA
<213> Artificial Sequence

<220>
<223> siRNA CD9 sens C

<400> 16
ggcaugaucu ucaguaugat t                                                21


<210> 17
<211> 21
<212> RNA
<213> Artificial Sequence

<220>
<223> siRNA CD9 antisens C

<400> 17
ucauacugaa gaucaugcct t                                                21